# EUROPEAN PATENT APPLICATION

(11) **EP 4 624 580 A1**
(43) Date of publication of application: **01.10.2025**
(21) Application number: 23901125.7
(22) Date of filing: 07.12.2023
(51) Int. Cl.: C12N 15/77, C12N 9/02, C12N 15/52, C12P 19/32

(54) **PURINE NUCLEOTIDE-PRODUCING MICROORGANISM AND METHOD FOR PRODUCTION OF PURINE NUCLEOTIDE USING SAME**

(30) Priority: 08.12.2022 KR 20220170770
(71) Applicant: CJ CheilJedang Corporation, Seoul 04560 (KR)
(72) Inventor: KWON, Hee Su, Seoul 04560 (KR); BONG, Hyunju, Seoul 04560 (KR); BAE, Hyun-jung, Seoul 04560 (KR); LEE, Ji Hye, Seoul 04560 (KR); KIM, Dae Young, Seoul 04560 (KR); LEE, Ji Hyun, Seoul 04560 (KR)
(74) Representative: Meissner Bolte Partnerschaft mbB
(86) International application number: PCT/KR2023/020105
(87) International publication number: WO 2024/123108

(57) **Abstract**

The present disclosure relates to a microorganism, in which the activity of formate dehydrogenase is weakened compared to the endogenous activity thereof; a method for producing purine nucleotides, comprising: culturing the microorganism in a medium; a composition for producing purine nucleotides, comprising the microorganism, a culture product of the microorganism, a fermented product of the microorganism, or a combination of two or more thereof; and use of the microorganism for the production of purine nucleotides.

## Description

### [Technical Field]

The present disclosure relates to a microorganism in which the activity of formate dehydrogenase is weakened compared to the endogenous activity thereof; a method for producing purine nucleotides, including: culturing the microorganism in a medium; a composition for producing purine nucleotides, including the microorganism, a culture product of the microorganism, a fermented product of the microorganism, or a combination of two or more thereof; and use of the microorganism for the production of purine nucleotides.

### [Background Art]

Purine nucleotides, *i.e.*, 5'-inosine monophosphate (hereinafter referred to as IMP), 5'-xanthosine monophosphate (hereinafter referred to as XMP), and 5'-guanosine monophosphate (hereinafter referred to as GMP), which are intermediates of the nucleic acid biosynthetic metabolic system, have an important physiological role in the body, and are widely used in foods, pharmaceuticals, *etc.* Specifically, IMP itself is known to impart a beef flavor, and GMP, which is derived from XMP, is known to impart a mushroom flavor. Both materials are known to enhance the taste intensity of monosodium glutamate (MSG), and thus have attracted much attention as a flavor-enriched nucleic acid-based seasoning.

Meanwhile, examples of the method for producing a purine nucleotide may include: (1) a method by enzymatic degradation of ribonucleic acid (RNA) extracted from yeast cells; (2) a method of preparation by fermentation by culturing a microorganism producing a purine nucleotide and directly recovering the purine nucleotide in the cultured solution; (3) a method by chemical phosphorylation of the nucleoside produced by fermentation; and (4) a method by enzymatic phosphorylation of the nucleoside produced by fermentation, *etc.* (KR Patent Publication No. 10-1049023, JP Patent Publication No. 4363042, KR Patent Publication No. 10-1210704, and Agri. Biol. Chem., 36(9), 1511-1522). Among these, while Method (1) has problems in terms of supply/demand of raw materials and economic efficiency, Method (2) is widely used due to its economic and environmental advantages. Meanwhile, in the case of the production of GMP, one of purine nucleotides, there is a disadvantage in that the yield is low due to its cell membrane permeability, and thus, a method of GMP production by enzymatic conversion of XMP produced through microbial fermentation is also utilized.

However, during the purine nucleotide production by fermentation using a microorganism, the microorganism may undergo stress due to temperature, pH, osmotic pressure, malnutrition, and oxidative factors. Among them, particularly in the oxidative stress, reactive oxygen species (ROS), which is an inevitable factor generated during the fermentative production, becomes the main cause, and the ROS may cause abnormal growth of the microorganism.

Accordingly, research for effective purine nucleotide production is still needed.

### [Disclosure]

### [Technical Problem]

The present inventors have developed a microorganism, in which the activity of formate dehydrogenase is weakened compared to the endogenous activity thereof; a method for producing purine nucleotides, including: culturing the microorganism in a medium; a composition for producing purine nucleotides, including the microorganism, a culture product of the microorganism, a fermented product of the microorganism, or a combination of two or more thereof; and use of the microorganism for the production of purine nucleotides, thereby completing the present disclosure.

### [Technical Solution]

It is one object of the present disclosure to provide a microorganism having a purine nucleotide producing ability, in which the activity of formate dehydrogenase is weakened compared to the endogenous activity thereof.

In one embodiment, the formate dehydrogenase may be composed of any one or more amino acid sequences selected from the group consisting of SEQ ID NO: 39, SEQ ID NO: 41, SEQ ID NO: 43, and a combination thereof.

In the microorganism according to any one of the above-described embodiments, the activity of any one or more proteins selected from the group consisting of (a) to (c) below may be further regulated:
(a) the activity of a glycine degradation enzyme is enhanced compared to the endogenous activity thereof;
(b) the activity of serine hydroxymethyltransferase is enhanced compared to the endogenous activity thereof; and
(c) the activity of formate-dependent phosphoribosylglycinamide formyltransferase is enhanced compared to the endogenous activity thereof.

In the microorganism according to any one of the above-described embodiments, the microorganism may be a microorganism of the genus *Corynebacterium.*

In the microorganism according to any one of the above-described embodiments, the microorganism of the genus *Corynebacterium* may be *Corynebacterium stationis.*

In the microorganism according to any one of the above-described embodiments, the microorganism may have an increased purine nucleotide producing ability compared to a non-modified microorganism.

It is another object of the present disclosure to provide a method for producing purine nucleotides, including: culturing a microorganism, in which the activity of formate dehydrogenase is weakened compared to the endogenous activity thereof, in a medium

In one embodiment, the method may further include recovering a target substance from the cultured microorganism, a culture product of the microorganism, a fermented product of the microorganism, or the culture medium.

It is still another object of the present disclosure to provide a composition for producing purine nucleotides: including a microorganism, in which the activity of formate dehydrogenase is weakened compared to the endogenous activity thereof, a culture product of the microorganism, a fermented product of the microorganism, or a combination of two or more thereof.

It is yet another object of the present disclosure to provide the use of a microorganism, in which the activity of formate dehydrogenase is weakened compared to the endogenous activity thereof, for the production of purine nucleotides.

### [Advantageous Effects]

The microorganism of the present disclosure, in which the activity of formate dehydrogenase is weakened compared to the endogenous activity thereof, can produce purine nucleotides in high yield and thus can be effectively used in the industrial production of purine nucleotides.

### [Detailed Description of Preferred Embodiments]

The present disclosure will be described in detail as follows. Meanwhile, each description and embodiment disclosed herein can be applied to other descriptions and embodiments, respectively. That is, all combinations of various elements disclosed herein fall within the scope of the present disclosure. Further, the scope of the present disclosure is not limited by the specific description described below.

Additionally, those of ordinary skill in the art may be able to recognize or confirm, using only conventional experimentation, many equivalents to the particular aspects of the invention described herein. Furthermore, it is also intended that these equivalents be included in the present disclosure.

As used in the specification and appended claims, the singular forms ("a", "an", and "the") include plural referents unless the context clearly states otherwise. Unless the context indicates otherwise, singular terms shall include the plural and plural terms shall include the singular. As used in the specification and appended claims, unless stated otherwise, the use of "or" may be used to include "and/or".

As used herein, the term "about" may be presented before a particular numerical value. The term "about" used herein includes not only the exact number recited after the term, but also a range that is near or close to that number. Considering the context in which the number is presented, it can be determined whether any number is close to or near the particular number presented. In one example, the term "about" may refer to a range from -10% to +10% of a numerical value. In another example, the term "about" may refer to a range from -5% to +5% of a given numerical value, but is not limited thereto.

As used herein, the descriptions such as the terms "first, second, third..." "i), ii), iii)..." or "(a), (b), (c), (d)..." are used to distinguish similar constitutions, and these terms do not mean that the constitutions are performed continually or sequentially. For example, when the terms are used in reference to steps of a method, use, or assay, there may be no time interval between these steps, or they may be performed concurrently, or may be performed several seconds, several minutes, several hours, several days, or several months apart.

As used herein, the term "consisting essentially of" may mean that, in the case where the features of the object claimed herein are not substantially affected by the presence of an unspecified component, the unspecified component may be present.

As used herein, the term, "consisting of" means that the total ratio of specific component(s) is 100%. The components or features that are recited after the term "consisting of" may be essential or mandatory. In some embodiments, in addition to the components or features recited after the term "consisting of", any other components or non-essential components may be excluded.

As used herein, the term "comprising/including" means the presence of features, steps or components recited after the term, and does not exclude the presence or addition of one or more features, steps or components. The components or features recited after the term "comprising/including" herein may be essential or mandatory. However, in some embodiments, the term may further include any other or non-essential components or features.

One aspect of the present disclosure provides a microorganism having a purine nucleotide producing ability, in which the activity of formate dehydrogenase is weakened compared to the endogenous activity thereof.

The weakened activity of the formate dehydrogenase may be defined as an increased purine nucleotide producing ability of the microorganism of the present disclosure compared to the production ability of a natural wild-type microorganism or non-modified microorganism (*e.g.*, a microorganism expressing a polypeptide having the activity of a wild-type formate dehydrogenase (*e.g*., any one or more polypeptides selected from the group consisting of SEQ ID NO: 39, SEQ ID NO: 41, SEQ ID NO: 43, and a combination thereof), or a strain in which the activity of formate dehydrogenase of the present disclosure is not weakened compared to the endogenous activity, or a strain before weakening thereof), but is not limited thereto.

For example, the activity of formate dehydrogenase may be evaluated by measuring the purine nucleotide producing ability or yield, but is not limited thereto.

As used herein, the term "formate dehydrogenase" refers to an enzyme, which is composed of formate dehydrogenase subunit 1 (Fdh subunit 1), formate dehydrogenase subunit 2 (Fdh subunit 2), and formate dehydrogenase subunit 3 (Fdh subunit 3), and which utilizes formic acid as a substrate and catalyzes an oxidation reaction to reduce NAD⁺ and produce NADH and CO₂. Specifically, the formate dehydrogenase of the present disclosure may be any one or more proteins selected from the group consisting of formate dehydrogenase subunit 1 (Fdh subunit 1), formate dehydrogenase subunit 2 (Fdh subunit 2), and forate dehydrogenase subunit 3 (Fdh subunit 3), but is not limited thereto. The formate dehydrogenase of the present disclosure may be used to refer to the complex of formate dehydrogenase subunit 1 (Fdh subunit 1), formate dehydrogenase subunit 2 (Fdh subunit 2), and formate dehydrogenase subunit 3 (Fdh subunit 3), and may be used interchangeably with Fdh.

As used herein, the "*fdh* gene", which is composed of the *fdh 1, fdh 2,* and *fdh* 3 genes, may be used to refer to a gene encoding the formate dehydrogenase of the present disclosure. Specifically, the *fdh* gene of the present disclosure may be used to refer to a gene encoding the complex of formate dehydrogenase subunit 1 (Fdh subunit 1), formate dehydrogenase subunit 2 (Fdh subunit 2), and formate dehydrogenase subunit 3 (Fdh subunit 3).

Specifically, the formate dehydrogenase of the present disclosure may be a protein having the activity of formate dehydrogenase subunit 1 (Fdh subunit 1), formate dehydrogenase subunit 2 (Fdh subunit 2), and formate dehydrogenase subunit 3 (Fdh subunit 3) encoded by the *fdh 1, fdh 2,* and *fdh* 3 genes, respectively, but the type thereof is not particularly limited as long as the protein has an activity corresponding to formate dehydrogenase. The formate dehydrogenase subunit 1 (Fdh subunit 1), formate dehydrogenase subunit 2 (Fdh subunit 2), and formate dehydrogenase subunit 3 (Fdh subunit 3) encoded by the *fdh 1, fdh 2,* and *fdh* 3 genes, respectively, are known in the art, and the amino acid and polynucleotide sequences of the formate dehydrogenase subunit 1 (Fdh subunit 1), formate dehydrogenase subunit 2 (Fdh subunit 2), and formate dehydrogenase subunit 3 (Fdh subunit 3) can be obtained from a known database, NCBI's GenBank. *etc.*, but is not limited thereto.

In one example, the formate dehydrogenase subunit 1 (Fdh subunit 1), formate dehydrogenase subunit 2 (Fdh subunit 2), and formate dehydrogenase subunit 3 (Fdh subunit 3) may include the amino acid sequences of SEQ ID NO: 39, SEQ ID NO: 41, and SEQ ID NO: 43, respectively, or amino acid sequences with at least 60% homology or identity thereto, but is not limited thereto as long as it has an activity of formate dehydrogenase. Specifically, polypeptides having the activity of formate dehydrogenase subunit 1 (Fdh subunit 1), formate dehydrogenase subunit 2 (Fdh subunit 2), and formate dehydrogenase subunit 3 (Fdh subunit 3) may have, include, consist of, or essentially consist of the amino acid sequences of SEQ ID NO: 39, SEQ ID NO: 41, and SEQ ID NO: 43, respectively, or amino acid sequences having at least 60%, 70%, 80%, 85%, 90%, 95%, 96%, 97%, 98%, or 99% or more homology or identity to SEQ ID NO: 39, SEQ ID NO: 41, and SEQ ID NO: 43, respectively. In one example, the formate dehydrogenase subunit 1 (Fdh subunit 1), formate dehydrogenase subunit 2 (Fdh subunit 2), and formate dehydrogenase subunit 3 (Fdh subunit 3) may refer to proteins endogenously present in microorganisms of the genus *Corynebacterium* or *Corynebacterium stationis,* but are not limited thereto. Specifically, the formate dehydrogenase subunit 1 (Fdh subunit 1), formate dehydrogenase subunit 2 (Fdh subunit 2), and formate dehydrogenase subunit 3 (Fdh subunit 3) may be formate dehydrogenase subunit 1 (Fdh subunit 1) consisting of the amino acid sequence of SEQ ID NO: 39, formate dehydrogenase subunit 2 (Fdh subunit 2) consisting of the amino acid sequence of SEQ ID NO: 41, and formate dehydrogenase subunit 3 (Fdh subunit 3) consisting of the amino acid sequence of SEQ ID NO: 43, respectively, which are endogenously present in microorganisms of the genus *Corynebacterium* or *Corynebacterium stationis,* but are not limited thereto.

With respect to amino acid sequences in the present disclosure, although it is described as a polypeptide "comprising/including" an amino acid sequence described by a specific sequence number, a polypeptide "consisting of" an amino acid sequence described by a specific sequence number, or a polypeptide or protein "having" an amino acid sequence described by a specific sequence number, it is apparent that any protein having an amino acid sequence in which part of the sequence is deleted, modified, substituted, conservatively substituted or added can be used in the present disclosure if it has the same or corresponding activity as the protein consisting of the amino acid sequence of the corresponding sequence number. For example, sequence additions upstream or downstream of the amino acid sequences that do not alter the function of the protein, naturally occurring mutations, silent mutation thereof, or conservative substitutions are not excluded, as long as the protein has activity identical or corresponding to the activity of the modified protein, and it will be apparent to those skilled in the art that such sequence additions or mutations belong to the scope of the present disclosure.

For example, it may be a case of sequence additions that do not alter the function of the variant polypeptide of the present disclosure, naturally occurring mutations, silent mutations thereof, or conservative substitutions at the N-terminus, C-terminus and/or within the amino acid sequences. For example, the polypeptides may be conjugated with a signal (or leader) sequence at the N-terminus involved in the translocation of proteins co-translationally or post-translationally. Further, the polypeptides may also be conjugated with another sequence or linker to identify, purify, or synthesize the polypeptides.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue. The amino acids may be classified into the following groups:
In one example, positively charged (basic) amino acids include arginine, lysine, and histidine; negatively charged (acidic) amino acids include glutamic acid and aspartic acid; amino acids having nonpolar side chains (nonpolar amino acids) include glycine, alanine, valine, leucine, isoleucine, methionine, phenylalanine, tryptophan, and proline; amino acids having polar or hydrophilic side chains (polar amino acids) include serine, threonine, cysteine, tyrosine, asparagine, and glutamine. In another example, amino acids having electrically charged side chain include arginine, lysine, histidine, glutamic acid, aspartate; and amino acids having uncharged amino acids (referred to as neutral amino acids) include glycine, alanine, valine, leucine, isoleucine, methionine, phenylalanine, tryptophan, proline, serine, threonine, cysteine, tyrosine, asparagine, and glutamine. In still another example, aromatic amino acids include phenylalanine, tryptophan, and tyrosine. In yet another example, branched amino acids include valine, leucine, and isoleucine. In even another example, the 20 amino acids can be classified according to their size into 5 groups, starting from amino acid groups with a relatively small volume, *i.e*., glycine, alanine, serine; cysteine, proline, threonine, aspartate, asparagine; valine, histidine, glutamic acid, glutamine; isoleucine, leucine, methionine, lysine, arginine; and phenylalanine, tryptophan, and tyrosine, but the classification of the amino acids is not limited thereto. Typically, conservative substitutions may have little or no effect on the activity of the polypeptide.

Additionally, the nucleotide sequence encoding the formate dehydrogenase may be a nucleotide sequence encoding a protein showing the activity of formate dehydrogenase.

For example, the nucleotide sequences encoding the formate dehydrogenase subunit 1 (Fdh subunit 1), formate dehydrogenase subunit 2 (Fdh subunit 2), and formate dehydrogenase subunit 3 (Fdh subunit 3) may be a nucleotide sequence encoding a protein showing the activity of formate dehydrogenase.

For example, the formate dehydrogenase subunit 1 (Fdh subunit 1), formate dehydrogenase subunit 2 (Fdh subunit 2), and formate dehydrogenase subunit 3 (Fdh subunit 3) having the amino acid sequences of SEQ ID NO: 39, SEQ ID NO: 41, and SEQ ID NO: 43, respectively, may be encoded by a polynucleotide that may have, include, consist of, essentially consist of nucleotide sequences of SEQ ID NO: 40, SEQ ID NO: 42, and SEQ ID NO: 44, respectively, or nucleotide sequences having 60% or more, 70% or more, 75% or more, 80% or more, 85% or more, 90% or more, 95% or more, 96% or more, 97% or more, 98% or more, and less than 100% homology or identity to the nucleotide sequences of SEQ ID NO: 40, SEQ ID NO: 42, and SEQ ID NO: 44, respectively, but are not limited thereto. In addition, the nucleotide sequences of SEQ ID NO: 40, SEQ ID NO: 42, and SEQ ID NO: 44 can be obtained from a known database, NCBI's GenBank, *etc.,* but are not limited thereto.

In the present disclosure, for example, the gene including the nucleotide sequence of SEQ ID NO: 40 may be used interchangeably with a polynucleotide including the nucleotide sequence of SEQ ID NO: 40, a gene or polynucleotide having the nucleotide sequence of SEQ ID NO: 40, and a gene or polynucleotide consisting of the nucleotide sequence of SEQ ID NO: 40.

The polynucleotide of the present disclosure may undergo various modifications in the coding region without changing the amino acid sequence of the formate dehydrogenase of the present disclosure, due to codon degeneracy or in consideration of the codons preferred in an organism in which the formate dehydrogenase of the present disclosure is to be expressed. Therefore, based on codon degeneracy, it is apparent that polynucleotides which may be translated into polypeptides consisting of the amino acid sequence of the formate dehydrogenase of the present disclosure or polypeptides having a homology or identity thereto may also be included. For example, the polynucleotide of the present disclosure may be SEQ ID NO: 40, SEQ ID NO: 42 and/or SEQ ID NO: 44, or a degenerated sequence thereof.

In another example, the polynucleotide of the present disclosure may have or include a nucleotide sequence having 60% or more, 70% or more, 75% or more, 80% or more, 85% or more, 90% or more, 95% or more, 96% or more, 97% or more, 98% or more, and less than 100% homology or identity to the nucleotide sequences of SEQ ID NO: 40, SEQ ID NO: 42 and/or SEQ ID NO: 44, or may consist or essentially consist of a nucleotide sequence having 60% or more, 70% or more, 75% or more, 80% or more, 85% or more, 90% or more, 95% or more, 96% or more, 97% or more, 98% or more, and less than 100% homology or identity to the nucleotide sequences of SEQ ID NO: 40, SEQ ID NO: 42 and/or SEQ ID NO: 44, but is not limited thereto.

Additionally, the polynucleotide of the present disclosure may include a probe that may be prepared from a known gene sequence, for example, any polynucleotide sequence which may hybridize with a sequence complementary to all or part of the polynucleotide sequence of the present disclosure under stringent conditions to encode the formate dehydrogenase of the present disclosure without limitation.

As used herein, the term "homology" or "identity" refers to the degree of relatedness between two given amino acid sequences or nucleotide sequences, and may be expressed as a percentage. The terms homology and identity may often be used interchangeably with each other.

The sequence homology or identity of conserved polynucleotides or polypeptides may be determined by standard alignment algorithms and may be used together with a default gap penalty established by the program being used. Substantially, homologous or identical sequences are generally expected to hybridize to all or at least about 50%, 60%, 70%, 80% or 90% of the entire length of the sequences under moderate or highly stringent conditions. Polynucleotides that contain degenerate codons instead of codons in the hybridizing polynucleotides may also be considered.

Whether any two polynucleotide or polypeptide sequences have homology, similarity, or identity may be determined by a known computer algorithm such as the "FASTA" program using default parameters as in Pearson et al. (1988) Proc. Natl. Acad. Sci. USA 85:2444. Alternatively, it may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277) (version 5.0.0 or later) (GCG program package (Devereux, J. et al., Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego, 1994; and CARILLO et al. (1988) SIAM J Applied Math 48:1073). For example, homology, similarity, or identity may be determined using BLAST or ClustalW of the National Center for Biotechnology Information.

The homology, similarity, or identity of polynucleotides or polypeptides may be determined by comparing sequence information using, for example, the GAP computer program, such as Needleman et al. (1970), J Mol Biol. 48:443, as disclosed in Smith and Waterman, Adv. Appl. Math (1981)2:482. In summary, the GAP program defines the homology, similarity or identity as the value obtained by dividing the number of similarly aligned symbols (*i.e*., nucleotides or amino acids) by the total number of the symbols in the shorter of the two sequences. Default parameters for the GAP program may include (1) a binary comparison matrix (containing a value of 1 for identities and 0 for non-identities) and the weighted comparison matrix (or EDNAFULL (EMBOSS version of NCBI NUC4.4) substitution matrix) of Gribskov et al. (1986) Nucl Acids Res. 14:6745, as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or a gap opening penalty of 10 and a gap extension penalty of 0.5); and (3) no penalty for end gaps.

Further, whether any two polynucleotide or polypeptide sequences have homology, similarity, or identity may be determined by comparing sequences via Southern hybridization experiments under defined stringent conditions, and the appropriate hybridization conditions to be defined may be determined by way of a method within the scope of the present disclosure, which is known to those skilled in the art (*e.g.*, J. Sambrook et al., Molecular Cloning, A Laboratory Manual, 2nd Edition, Cold Spring Harbor Laboratory press, Cold Spring Harbor, N.Y., 1989; F. M. Ausubel et al., Current Protocols in Molecular Biology, John Wiley & Sons, Inc., New York), but is not limited thereto.

The "stringent conditions" refer to conditions which allow the specific hybridization between polynucleotides. Such conditions are disclosed in detail in the literature (*e.g.*, J. Sambrook *et al.*, *supra*)*.* For example, the stringent conditions may include conditions under which polynucleotides having high homology or identity, polynucleotides having 60% or more, 70% or more, 75% or more, 80% or more, 85% or more, 90% or more, 95% or more, 96% or more, 97% or more, 98% or more, or 99% or more hybridize with each other, while polynucleotides having homology or identity lower than the above homology or identity do not hybridize with each other; or may include ordinary washing conditions of Southern hybridization, *i.e.*, washing once, specifically twice or three times, at a salt concentration and a temperature corresponding to 60°C, 1×SSC, 0.1% SDS, specifically 60°C, 0.1×SSC, 0.1% SDS, and more specifically 68°C, 0.1×SSC, 0.1% SSD.

Hybridization requires that two nucleic acids have complementary sequences, although mismatches between bases are possible depending on the stringency of the hybridization. The term "complementary" is used to describe a relationship between nucleotide bases that may hybridize with each other. For example, with respect to DNA, adenine is complementary to thymine, and cytosine is complementary to guanine. Therefore, the present disclosure may also include an isolated nucleic acid fragment complementary to the entire sequence as well as a nucleic sequence substantially similar thereto.

For example, polynucleotides having a homology or identity to the polynucleotide of the present disclosure may be detected using the hybridization conditions including a hybridization step at a Tₘ value of 55°C under the above-described conditions. Further, the Tₘ value may be 60°C, 63°C, or 65°C, but is not limited thereto, and may be appropriately adjusted by those skilled in the art depending on the purpose thereof.

The appropriate stringency for hybridizing polynucleotides depends on the length and degree of complementarity of the polynucleotides, and these variables are well known in the art (see Sambrook *et al.*, *supra*, 9.50-9.51, 11.7-11.8).

As used herein, the term "purine nucleotide" may refer to specifically any one or more nucleotides selected from the group consisting of 5'-inosine monophosphate (hereinafter referred to as IMP), 5'-xanthosine monophosphate (hereinafter referred to as XMP), and 5'-guanosine monophosphate (hereinafter referred to as GMP). The IMP is a deaminated adenine compound, and refers to a nucleotide composed of one molecule each of hypoxanthine, ribose, and phosphate. The IMP may be biosynthesized from 5'-phosphoribosyl-1-pyrophosphate (PRPP), and specifically, it may be formed by substituting a pyrophosphate group bound to the first carbon of PRPP with a nitrogen atom and by constituting an imidazole ring and a pyrimidine ring via nine stages. The XMP refers to a nucleotide resulting from dehydrogenation of IMP, and may be synthesized from IMP by inosine-5'-monophosphate dehydrogenase. The GMP refers to a nucleotide having a structure in which a phosphate group forms an ester bond in a ribose of a guanosine molecule. The GMP may be synthesized by adding ammonia molecules to XMP via 5'-guanylic acid biosynthase (GMP synthase). A method of preparing GMP from XMP and/or a means used in the method may be selected from known techniques.

As used herein, the term "microorganism (or strain)" includes all wild-type microorganisms, or naturally or artificially genetically modified microorganisms, and it may be a microorganism in which a particular mechanism is weakened or enhanced due to insertion of a foreign gene, or enhancement or inactivation of the activity of an endogenous gene, *etc.,* and may be a microorganism including genetic modification to produce a target polypeptide, protein or product. In the present disclosure, the "microorganism" and "strain" have the same meaning and may be used interchangeably without limitation.

For example, the microorganism of the present disclosure may be a microorganism (*e.g*., a recombinant strain) in which the activity of the formate dehydrogenase is weakened compared to the endogenous activity, but is not limited thereto.

As used herein, the term "microorganism having a purine nucleotide producing ability", which is a prokaryotic or eukaryotic microbial strain capable of producing a purine nucleotide in an organism, may include all microorganisms, in which a purine nucleotide producing ability has been imparted to a parent strain without a purine nucleotide producing ability, or microorganisms that endogenously have a purine nucleotide producing ability. The purine nucleotide producing ability may be imparted or enhanced by improvement of species.

As used herein, the term "non-modified microorganism" does not exclude a strain containing a mutation that may occur naturally in a microorganism, and may refer to a wild-type strain or natural-type strain itself, or a strain before its trait is altered due to genetic modification caused by natural or artificial factors. The "non-modified microorganism" may be used interchangeably with "strain before modification", "microorganism before modification", "non-mutant strain", "non-modified strain", "non-mutant microorganism", "parent strain before mutation", "wild-type microorganism", "reference microorganism", or "standard microorganism". In the present disclosure, the non-modified microorganism may refer to a strain in which the activity of formate dehydrogenase of the present disclosure is not weakened compared to the endogenous activity, or a strain before weakening thereof, but is not limited thereto. In addition, in the present disclosure, the non-modified microorganism may be a microorganism including the amino acid sequences consisting of SEQ ID NO: 39, SEQ ID NO: 41, and/or SEQ ID NO: 43, or the polynucleotides consisting of SEQ ID NO: 40, SEQ ID NO: 42, and/or SEQ ID NO: 44, but is not limited thereto.

For the purpose of the present disclosure, the microorganism of the present disclosure may include all microorganisms capable of producing the target purine nucleotides by weakening the activity of formate dehydrogenase compared to the endogenous activity. For example, the microorganism of the present disclosure is characterized in that the activity of formate dehydrogenase is weakened compared to the endogenous activity, thereby increasing purine nucleotide producing ability, and may be a genetically modified microorganism or a recombinant microorganism, but is not limited thereto. Specifically, the recombinant strain with an increased purine nucleotide producing ability may be a microorganism having an increased purine nucleotide producing ability compared to a natural wild-type microorganism or a non-modified microorganism having an endogenous activity of formate dehydrogenase, but is not limited thereto.

In one example, the microorganism having a purine nucleotide producing ability, which is a prokaryotic or eukaryotic microbial strain capable of producing a purine nucleotide in an organism, may include all microorganisms that endogenously have a purine nucleotide producing ability, or microorganisms, in which a purine nucleotide producing ability has been imparted to a parent strain without a purine nucleotide producing ability by weakening the activity of formate dehydrogenase. The purine nucleotide producing ability may be imparted or enhanced by improvement of species.

The "endogenous activity" refers to the activity of a particular polypeptide originally possessed by a parent strain before transformation or a non-modified microorganism when a trait is altered by genetic variation due to a natural or artificial factor. The endogenous activity may also be interchangeably used with "activity before modification".

As used herein, the term "weakening" of a polypeptide activity is a comprehensive concept including both reduced or no activity compared to its endogenous activity. The weakening may be used interchangeably with terms such as deficiency, inactivation, deletion, disruption, down-regulation, decrease, attenuation, repression, reduction, *etc.*

For example, the weakening may refer to a state in which a protein exhibits activity but is not completely inactivated by deletion, and may mean that the activity of the protein is weakened compared to that of a non-modified microorganism, a wild-type strain, or a parent strain, but is not limited thereto.

For example, the weakening may be, but is not limited to, inactivation. The inactivation may mean that the protein consisting of any one or more amino acid sequences selected from the group consisting of SEQ ID NO: 39, SEQ ID NO: 41, SEQ ID NO: 43, and a combination thereof is not expressed at all, or the activity thereof is not exhibited or weakened even when the protein is expressed, as compared to a parent strain or a non-modified strain.

The weakening may also include a case where the polypeptide activity itself is decreased or removed compared to the activity of the polypeptide originally possessed by a microorganism due to a mutation of the polynucleotide encoding the polypeptide; a case where the overall level of intracellular polypeptide activity is decreased compared to a natural strain due to the inhibition of expression of the gene encoding the same, or the inhibition of translation into the polypeptide, *etc.;* a case where the gene is not expressed at all; and a case where no polypeptide activity is observed even when the gene is expressed.

The weakening of the activity of the polypeptide compared to its endogenous activity means that the activity of the polypeptide is decreased compared to the activity of a particular polypeptide originally possessed by a parent strain before modification or a non-modified microorganism. Whether or not the activity of the polypeptide is weakened may be confirmed from the activity level of the corresponding polypeptide, expression level thereof, or the decrease in the amount of products produced from the corresponding polypeptide.

In one example, the weakening may mean that the activity of formate dehydrogenase is less than about 100%, about 90% or less, about 80% or less, about 70% or less, about 60% or less, about 50% or less, about 40% or less, about 30% or less, about 20% or less, about 10% or less, about 5% or less, or 0%, relative to the activity of formate dehydrogenase of a parent strain before modification or a non-modified microorganism, but is not limited thereto.

For example, the inactivation may mean that the formate dehydrogenase is not expressed at all, or the activity thereof is not exhibited or weakened even when the formate dehydrogenase is expressed, compared to a non-modified microorganism,

The weakening of the polypeptide activity can be performed by any method known in the art, but the method is not limited thereto, and can be achieved by applying various methods well known in the art (*e.g*., Nakashima N et al., Bacterial cellular engineering by genome editing and gene silencing. Int J Mol Sci. 2014;15(2):2773-2793; Sambrook et al. Molecular Cloning 2012; *etc.*).

Specifically, the weakening of the polypeptide activity of the present disclosure may be achieved by:
1) deleting a part or all of the gene encoding the polypeptide;
2) modifying the expression regulatory region (expression regulatory sequence) such that the expression of the gene encoding the polypeptide is decreased;
3) modifying the amino acid sequence constituting the polypeptide such that the polypeptide activity is removed or weakened (*e.g*., deletion/substitution/addition of one or more amino acids on the amino acid sequence);
4) modifying the polynucleotide sequence encoding the polypeptide such that the polypeptide activity is removed or weakened (*e.g*., deletion/substitution/addition of one or more of nucleotides on the nucleotide sequence of the polypeptide gene to encode a polypeptide that has been modified to remove or weaken the activity of the polypeptide);
5) modifying the nucleotide sequence encoding the initiation codon or 5'-UTR of the gene encoding the polypeptide;
6) introducing an antisense oligonucleotide (*e.g*., antisense RNA), which binds complementary to the gene transcript encoding the polypeptide;
7) adding a sequence complementary to the Shine-Dalgarno (SD) sequence on the front end of the SD sequence of the gene encoding the polypeptide to form a secondary structure, thereby inhibiting the ribosomal attachment;
8) a reverse transcription engineering (RTE), which adds a promoter, which is to be reversely transcribed, on the 3' terminus of the open reading frame (ORF) of the polynucleotide sequence encoding the polypeptide; or
9) a combination of two or more selected from the methods 1) to 8) above, but is not particularly limited thereto.

For example,
The 1) method of deleting a part or all of the gene encoding the polypeptide may be achieved by deleting all of the polynucleotide encoding the endogenous target polypeptide within the chromosome, or by replacing the polynucleotide with a polynucleotide or a marker gene having partially deleted nucleotides.

The method of deleting a part or all of the polynucleotide may be achieved by a method for deleting the polynucleotide by homologous recombination through a vector for chromosomal insertion in the microorganism, or a method, in which light, such as UV rays, or a chemical substance may be used to induce mutations, and a target gene-deleted strain may be selected from the resulting mutants, but is not limited thereto. The method of deleting a part or all of the gene may include a method using DNA recombination technology. For example, a part or all of the gene may be deleted by injecting a nucleotide sequence or a vector containing a nucleotide sequence homologous to the target gene into the microorganism to induce homologous recombination. The injected nucleotide sequence or the vector may include a dominant selection marker, but is not limited thereto.

The 2) method of modifying the expression regulatory region may be achieved by inducing a modification on the expression regulatory region (expression regulatory sequence) through deletion, insertion, non-conservative substitution or conservative substitution, or a combination thereof; or by replacing the sequence with a sequence having a weaker activity. The expression regulatory region may include a promoter, an operator sequence, a sequence encoding a ribosome binding site, and a sequence for regulating transcription and translation, but is not limited thereto.

The 3) and 4) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to weaken the activity of the polypeptide, or by replacing the sequence with an amino acid sequence or a polynucleotide sequence modified to have a weaker activity, or an amino acid sequence or a polynucleotide sequence modified to have no activity, but are not limited thereto. For example, the expression of a gene may be inhibited or weakened by introducing a modification into the polynucleotide sequence to form a stop codon, but is not limited thereto

The 5) method of modifying the nucleotide sequence encoding the initiation codon or 5'-UTR of the gene encoding the polypeptide may be achieved, for example, by substituting the nucleotide sequence with a nucleotide sequence encoding another initiation codon having a lower polypeptide expression rate than the endogenous initiation codon, but is not limited thereto.

The 6) method of introducing an antisense oligonucleotide (*e.g*., antisense RNA), which binds complementary to the gene transcript encoding the polypeptide can be found in the literature (Weintraub, H. et al., Antisense-RNA as a molecular tool for genetic analysis, Reviews-Trends in Genetics, Vol. 1(1) 1986).

The 7) method of adding a sequence complementary to the Shine-Dalgarno (SD) sequence on the front end of the SD sequence of the gene encoding the polypeptide to form a secondary structure, thereby inhibiting the ribosome attachment may be achieved by inhibiting mRNA translation or reducing the speed thereof.

The 8) reverse transcription engineering (RTE), which adds a promoter, which is to be reversely transcribed, on the 3' terminus of the open reading frame (ORF) of the gene sequence encoding the polypeptide may be achieved by forming an antisense nucleotide complementary to the gene transcript encoding the polypeptide to weaken the activity.

The modification of a part or all of the polynucleotide in the microorganism of the present disclosure may be achieved by (a) homologous recombination through a vector for chromosomal insertion in the microorganism or genome editing using an engineered nuclease (*e.g*., CRISPR-Cas9), and/or (b) may be induced by light, such as ultraviolet rays and radiation, *etc.* and/or chemical treatments, but is not limited thereto. The method of modifying a part or all of the gene may include a method using DNA recombination technology. For example, a part or all of the gene may be deleted by injecting a nucleotide sequence or a vector containing a nucleotide sequence homologous to the target gene into the microorganism to induce homologous recombination. The injected nucleotide sequence or the vector may include a dominant selection marker, but is not limited thereto.

In one example, the recombinant microorganism having a purine nucleotide producing ability of the present disclosure may include all microorganisms capable of producing purine nucleotides, which are transformed through a vector to weaken the activity of formate dehydrogenase of the present disclosure.

The vector of the present disclosure may include a DNA construct containing the nucleotide sequence of a polynucleotide encoding the target polypeptide operably linked to a suitable expression regulatory region (expression regulatory sequence) so as to be able to express the target polypeptide in a suitable host cell. The expression regulatory region may include a promoter capable of initiating transcription, any operator sequence for regulating the transcription, a sequence encoding a suitable mRNA ribosome binding site, and a sequence for regulating termination of transcription and translation. Once transformed into a suitable host cell, the vector may replicate or function independently from the host genome, or may integrate into genome thereof.

The vector used in the present disclosure is not particularly limited, and any vector known in the art may be used. Examples of the vector typically used may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, *etc.* may be used; and as a plasmid vector, those based on pDZ, pBR, pUC, pBluescriptII, pGEM, pTZ, pCL, pSK, pSKH and pET, *etc.* may be used. Specifically, pDZ, pDC, pDCM2, pACYC177, pACYC184, pCL, pSK, pSKH130, pECCG117, pUC19, pBR322, pMW118, pCC1BAC vector, *etc.* may be used

In one example, a polynucleotide encoding a target polypeptide may be inserted into the chromosome through a vector for intracellular chromosomal insertion. The insertion of the polynucleotide into the chromosome may be performed by any method known in the art, for example, by homologous recombination, but is not limited thereto. The vector may further include a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cell toxic agents, or expression of surface polypeptides, may be used. Only cells expressing the selection marker are able to survive or to show different phenotypes under the environment treated with the selective agent, and thus the transformed cells may be selected.

As used herein, the term "transformation" refers to the introduction of a vector containing a polynucleotide encoding a target polypeptide into a host cell or microorganism so that the polypeptide encoded by the polynucleotide can be expressed in the host cell. As long as the transformed polynucleotide can be expressed in the host cell, it does not matter whether the transformed polynucleotide is integrated into the chromosome of the host cell and located therein or located extrachromosomally, and both cases can be included. Further, the polynucleotide may include DNA and/or RNA encoding the target polypeptide. The polynucleotide may be introduced in any form, as long as it can be introduced into the host cell and expressed therein. For example, the polynucleotide may be introduced into the host cell in the form of an expression cassette, which is a gene construct including all elements required for its autonomous expression. The expression cassette may commonly include a promoter operably linked to the polynucleotide, a transcription terminator, a ribosome binding site, or a translation terminator. The expression cassette may be in the form of a self-replicable expression vector. Additionally, the polynucleotide may be introduced into a host cell as it is and operably linked to sequences required for expression in the host cell, but is not limited thereto.

As used herein, the term "operably linked" refers to a constitution of placing a regulatory sequence to an appropriate position to regulate expression of a coding sequence. Thus, the term "operably linked" includes an attachment or linking between a regulatory region of a functional domain having a known or desired activity such as a promoter, a stop codon, a signal sequence, or an enhancer, and a target (gene or polypeptide) such that the expression, secretion, or function of the target may be regulated in accordance with the known or desired activity. For example, it may mean that the polynucleotide sequence is functionally linked to a promoter sequence that initiates and mediates transcription of the polynucleotide encoding the target variant polypeptide of the present disclosure.

As used herein, the term "expression" includes any step involved in the production of a polypeptide, *e.g*., transcription, post-transcriptional modification, translation, post-translational modification, and secretion, *etc.,* but is not limited thereto.

As used herein, the term "expression vector" refers to a linear or circular nucleic acid molecule including a coding sequence and an operably linked regulatory sequence for expression thereof.

As used herein, the term "regulatory sequence" refers to a polynucleotide sequence necessary for the expression of a coding sequence. Each regulatory sequence may be native (derived from the same origin) or foreign (derived from different genes) to the coding sequence. Examples of the regulatory sequence may include a leader sequence, a polyadenylation sequence, a pro-peptide sequence, a promoter, a signal peptide sequence, an operator sequence, a sequence encoding a ribosome binding site, and a sequence for regulating the termination of transcription and translation. The minimum units of the regulatory sequence may include a promoter, and a sequence for terminating transcription and translation.

As used herein, the term "recombinant" with respect to a cell, polynucleotide, polypeptide, or vector, indicates that the cell, polynucleotide, polypeptide or vector has been modified by the introduction of a heterologous nucleic acid or polypeptide or the alteration of a native polynucleotide or polypeptide, or that the cell is derived from a cell thus modified. Therefore, for example, recombinant cells may express genes that are not found within the native (non-recombinant) form of the cells, or may express native genes that are otherwise abnormally expressed, under expressed or not expressed at all.

For example, the microorganism for producing a purine nucleotide may be a microorganism endogenously including a protein consisting of the amino acid sequences of SEQ ID NO: 39, SEQ ID NO: 41, and/or SEQ ID NO: 43, or a protein consisting of amino acid sequences having at least 60%, 70%, 75%, 80%, 85%, 90%, 95%, 96%, 97%, 98%, 99%, 99.5%, 99.7%, or 99.9% or more homology or identity to SEQ ID NO: 39, SEQ ID NO: 41, and/or SEQ ID NO: 43.

For example, the microorganism for producing a purine nucleotide may be a microorganism endogenously including a polynucleotide sequence capable of encoding a protein including amino acid sequences having at least 80% homology to SEQ ID NO: 39, SEQ ID NO: 41 and/or SEQ ID NO: 43, the nucleotide sequences of SEQ ID NO: 40, SEQ ID NO: 42 and/or SEQ ID NO: 44, and nucleotide sequences having 60% or more, 70% or more, 75% or more, 80% or more, 85% or more, 90% or more, 95% or more, 96% or more, 97% or more, 98% or more, and less than 100% homology or identity to the nucleotide sequences of SEQ ID NO: 40, SEQ ID NO: 42, and/or SEQ ID NO: 44.

The microorganism of the present disclosure may include all microorganisms, in which the activity of formate dehydrogenase is weakened compared to the endogenous activity thereof by various methods known in the art.

In one embodiment, the microorganism of the present disclosure, in which the activity of formate dehydrogenase is weakened compared to the endogenous activity thereof, may be a microorganism in which the polynucleotide encoding formate dehydrogenase is deleted or modified, but is not limited thereto. Specifically, it may be a microorganism having no activity of formate dehydrogenase due to deletion of the nucleotide sequences of SEQ ID NO: 40, SEQ ID NO: 42, and SEQ ID NO: 44, but is not limited thereto.

In one example, the microorganism with an increased purine nucleotide producing ability of the present disclosure may be a microorganism with an increased purine nucleotide producing ability compared to a non-modified microorganism, but is not limited thereto. In one example, the non-modified microorganism that is the target strain for comparing the increase in purine nucleotide producing ability may be CJX1664 strain or KCCM12151P strain, but is not limited thereto.

In one example, the microorganism with an increased purine nucleotide producing ability may have an increased purine nucleotide producing ability by about 1% or more, specifically about 1% or more, about 2.5% or more, about 5% or more, about 6% or more, about 7% or more, about 8% or more, about 9% or more, about 10% or more, about 15% or more, about 20% or more, about 25% or more, about 30% or more, or about 35% or more (the upper limit is not particularly limited, for example, about 200% or less, about 150% or less, about 100% or less, about 50% or less, about 45% or less, or about 40% or less), as compared to that of a parent strain before modification or a non-modified microorganism, but is not limited thereto, as long as it has an increased + value compared to the production ability of a parent strain before modification or non-modified microorganism. In another example, the microorganism with an increased purine nucleotide producing ability may have an increased purine nucleotide producing ability by about 1.1 times or more, about 1.15 times or more, about 1.2 times or more, about 1.25 times or more, about 1.3 times or more, or about 1.35 times or more (the upper limit is not particularly limited, for example, about 10 times or less, about 5 times or less, about 3 times or less, about 2 times or less, about 1.5 times or less, or about 1.4 times or less) as compared to that of a parent strain before modification or a non-modified microorganism, but is not limited thereto.

In one example, the microorganism having a purine nucleotide producing ability may be a prokaryotic cell or eukaryotic cell, but may specifically be a prokaryotic cell. The prokaryotic cell may include, for example, a microbial strain belonging to the genus *Escherichia*, the genus *Erwinia*, the genus *Serratia*, the genus *Providencia*, the genus *Corynebacterium,* the genus *Pseudomonas*, the genus *Leptospira*, the genus *Salmonella*, the genus *Brevibacteria*, the genus *Hypomononas*, the genus *Chromobacterium*, and the genus *Norcardia*, or fungi or yeasts. Specifically, the microorganism may be a microbial strain belonging to the genus *Escherichia*, the genus *Corynebacterium,* the genus *Leptospira*, and yeasts. More specifically, it may be a microbial strain belonging to the genus *Corynebacterium.*

In the microorganism according to any one of the above-described embodiments, the microorganism of the present disclosure may be a microorganism belonging to the genus *Corynebacterium.*

In one example, the microorganism of the present disclosure may be *Corynebacterium stationis, Corynebacterium glutamicum, Corynebacterium crudilactis, Corynebacterium deserti, Corynebacterium efficiens, Corynebacterium callunae, Corynebacterium singulare*, *Corynebacterium halotolerans, Corynebacterium striatum, Corynebacterium ammoniagenes, Corynebacterium pollutisoli*, *Corynebacterium imitans*, *Corynebacterium testudinoris,* or *Corynebacterium flavescens.* Specifically, the microorganism of the present disclosure may be a microorganism belonging to the genus *Corynebacterium,* more specifically, *Corynebacterium stationis* or *Corynebacterium glutamicum*, but is not limited thereto.

The microorganism having a purine nucleotide producing ability may be a microorganism having an incased purine nucleotide producing ability by further regulating the activity of any one or more proteins selected from the group consisting of (a) to (c) below, but is not limited thereto:
(a) the activity of a glycine degradation enzyme is enhanced compared to the endogenous activity thereof;
(b) the activity of serine hydroxymethyltransferase is enhanced compared to the endogenous activity thereof; and
(c) the activity of formate-dependent phosphoribosylglycinamide formyltransferase is enhanced compared to the endogenous activity thereof.

In the microorganism according to any one of the above-described embodiments, the microorganism having a purine nucleotide producing ability of the present disclosure may be a microorganism having an incased purine nucleotide producing ability by further regulating the activity of any one or more proteins selected from the group consisting of (a) to (d) below, but is not limited thereto:
(a) the activity of a glycine degradation enzyme is enhanced compared to the endogenous activity thereof;
(b) the activity of serine hydroxymethyltransferase is enhanced compared to the endogenous activity thereof;
(c) the activity of formate-dependent phosphoribosylglycinamide formyltransferase is enhanced compared to the endogenous activity thereof; and
(d) the activity of serine dehydratase is weakened compared to the endogenous activity thereof.

As used herein, the term "enhancement" of polypeptide activity means that the activity of a polypeptide is increased, as compared with its endogenous activity thereof. The enhancement may be used interchangeably with terms such as activation, up-regulation, overexpression, increase, *etc.*

The enhancement may include both cases in which an activity not originally possessed is exhibited, or an activity is enhanced compared to the endogenous activity or the activity before modification.

For example, the term "activity not originally possessed is exhibited" may refer to the "introduction of a protein", but is not limited thereto. The introduction of a protein means that a gene not originally possessed by a microorganism is expressed in the microorganism and thus the microorganism exhibits the activity of a particular protein or exhibits increased or enhanced activity of the corresponding protein compared with the endogenous activity or the activity before modification. For example, the term may indicate that a polynucleotide encoding a particular protein is introduced into the chromosome of a microorganism, or a vector containing a polynucleotide encoding a particular protein is introduced into a microorganism, thereby exhibiting the activity of the particular protein.

The "endogenous activity" refers to the activity of a particular polypeptide originally possessed by a parent strain before transformation or a non-modified microorganism when a trait is altered by genetic variation due to a natural or artificial factor. The endogenous activity may also be interchangeably used with "activity before modification".

The enhancement in the activity of the polypeptide, as compared with the endogenous activity, means that the activity of the polypeptide is enhanced, as compared with the activity and/or concentration (expression level) of a particular polypeptide originally possessed by a parent strain before transformation or a non-modified microorganism.

In one example, the enhancement may mean that the activity of a corresponding protein not originally possessed is exhibited, or the activity or concentration thereof is enhanced generally by about 1%, about 10%, about 25%, about 50%, about 75%, about 100%, about 150%, about 200%, about 300%, about 400% or about 500%, maximum about 1000% or about 2000% or more, based on the activity or concentration of a wild-type protein or an initial microbial strain, but is not limited thereto.

The enhancement of the activity of the polypeptide may be achieved by introducing a foreign polypeptide or enhancing the activity of an endogenous polypeptide. Whether or not the activity of the polypeptide is enhanced may be confirmed from the activity level of the corresponding polypeptide, expression level thereof, or the increase in the amount of products produced from the corresponding polypeptide.

The enhancement of the activity of the polypeptide may be applied by various methods well known in the art, and is not limited as long as it can enhance the activity of the target polypeptide compared to that of the microorganism before modification. Specifically, genetic engineering and/or protein engineering well known to those skilled in the art, which is a common method of molecular biology, may be used, but the method is not limited thereto (*e.g.*, Sitnicka et al. Functional Analysis of Genes. Advances in Cell Biology. 2010, Vol. 2. 1-16; Sambrook et al. Molecular Cloning 2012; *etc.*).

Specifically, the enhancement of the polypeptide activity of the present disclosure may be achieved by:
1) increasing the intracellular copy number of a polynucleotide encoding the polypeptide;
2) modifying the expression regulatory region of a gene encoding the polypeptide on the chromosome (*e.g*., inducing a modification within the expression regulatory region, replacing with a sequence having a stronger activity, or inserting a sequence having a stronger activity);
3) modifying the nucleotide sequence encoding the initiation codon or 5'-UTR of the gene transcript encoding the polypeptide;
4) modifying the amino acid sequence of the polypeptide such that the activity of the polypeptide is enhanced;
5) modifying the polynucleotide sequence encoding the polypeptide such that the activity of the polypeptide is enhanced (*e.g*., modifying the polynucleotide sequence of the polypeptide gene to encode a polypeptide that has been modified to enhance the activity of the polypeptide);
6) introducing a foreign polypeptide exhibiting the polypeptide activity or a foreign polynucleotide encoding the same;
7) codon-optimization of a polynucleotide encoding the polypeptide;
8) analyzing the tertiary structure of the polypeptide and thereby selecting and modifying the exposed site, or chemically modifying the same; or
9) a combination of two or more selected from above 1 to 8), but is not particularly limited thereto.

For example,
The 1) method of increasing the intracellular copy number of a polynucleotide encoding the polypeptide may be achieved by introducing a vector, which is operably linked to the polynucleotide encoding the polypeptide and is able to replicate and function regardless of a host cell, into the host cell. Alternatively, the method may be achieved by introducing one copy or two copies of polynucleotides encoding the polypeptide into the chromosome of a host cell. The introduction into the chromosome may be performed by introducing a vector, which is able to insert the polynucleotide into the chromosome of a host cell, into the host cell, but is not limited thereto. The vector is as described above.

The 2) method of replacing the expression regulatory region (or expression regulatory sequence) of a gene encoding the polypeptide on the chromosome with a sequence having a strong activity may be achieved, for example, by inducing a modification on the sequence through deletion, insertion, non-conservative or conservative substitution, or a combination thereof to further enhance the activity of the expression regulatory region, or by replacing the sequence with a sequence having a stronger activity. The expression regulatory region may include, but is not particularly limited to, a promoter, an operator sequence, a sequence encoding a ribosome binding site, and a sequence regulating the termination of transcription and translation, *etc.* In one example, the method may include replacing the original promoter with a strong promoter, but is not limited thereto.

Examples of the known strong promoter may include cj1 to cj7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, *etc.,* but the strong promoter is not limited thereto.

The 3) method of modifying the nucleotide sequence encoding the initiation codon or 5'-UTR of the gene encoding the polypeptide may be achieved, for example, by substituting the nucleotide sequence with a nucleotide sequence encoding another initiation codon having a higher expression rate of the polypeptide compared to the endogenous initiation codon, but is not limited thereto.

The 4) and 5) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to enhance the activity of the polypeptide, or by replacing the sequence with an amino acid sequence or a polynucleotide sequence modified to have a stronger activity, or an amino acid sequence or a polynucleotide sequence modified to enhance the activity, but are not limited thereto. The replacement may specifically be performed by inserting the polynucleotide into the chromosome by homologous recombination, but is not limited thereto. The vector used herein may further include a selection marker to confirm the insertion into the chromosome. The selection marker is as described above.

The 6) method of introducing a foreign polynucleotide exhibiting the activity of the polypeptide may be achieved by introducing into a host cell a foreign polynucleotide encoding a polypeptide that exhibits the same/similar activity to that of the polypeptide. The foreign polynucleotide may be used without limitation regardless of its origin or sequence as long as it exhibits the same/similar activity to that of the polypeptide. The introduction may be performed by those of ordinary skill in the art by appropriately selecting a transformation method known in the art, and the expression of the introduced polynucleotide in the host cell enables to produce the polypeptide, thereby increasing its activity.

The 7) method of codon-optimization of the polynucleotide encoding the polypeptide may be achieved by codon-optimization of an endogenous polynucleotide to increase the transcription or translation within a host cell, or by optimizing the codons thereof such that the optimized transcription and translation of the foreign polynucleotide can be achieved within the host cell.

The 8) method of analyzing the tertiary structure of the polypeptide and thereby selecting and modifying the exposed site, or chemically modifying the same may be achieved, for example, by comparing the sequence information of the polypeptide to be analyzed with a database, in which the sequence information of known proteins is stored, to determine template protein candidates according to the degree of sequence similarity, and thus confirming the structure based on the information, thereby selecting and transforming or modifying the exposed site to be modified or chemically modified.

Such enhancement of the polypeptide activity may mean that the activity or concentration of the corresponding polypeptide is increased relative to the activity or concentration of a polypeptide expressed in a wild-type strain or a microorganism before modification, or that the amount of products produced from the corresponding polypeptide is increased, but is not limited thereto.

In one example, the microorganism having a purine nucleotide producing ability of the present disclosure may be a microorganism with an enhanced purine nucleotide producing ability by further enhancing the activity of glycine degradation enzyme compared to the endogenous activity thereof, but is not limited thereto.

As used herein, the term "glycine degradation enzyme" is a protein that is directly or indirectly involved in the glycine degradation pathway, and may be used to mean each protein constituting the glycine degradation system, or the complex of the proteins, or the glycine degradation system itself. Specifically, the glycine degradation enzyme may be any one or more selected from the group consisting of T-protein (GcvT), P-protein (GcvP), L-protein (GcvL), H-protein (GcvH) that constitute the glycine degradation system, and LipB or LipA, which are coenzymes of the glycine degradation system, but is not limited thereto (John E. Cronan, Microbiology and Molecular Biology Reviews, 13 Apr. 2016). The glycine degradation enzyme of the present disclosure may be used interchangeably with GcvPTH or glycine cleavage system.

As used herein, the "glycine cleavage system (GCV)" is composed of two or more subunits, i.e., the subunits GcvP, GcvT, and GcvH, which takes the form of a multi-enzyme complex, which catalyzes the oxidative decarboxylation and deamination of glycine to carbon dioxide, ammonium ions and N⁵⁻¹⁰-methylenetetrahydrofolate. The glycine cleavage system of the present disclosure may be used interchangeably with the glycine degradation system.

Specifically, the glycine degradation enzyme of the present disclosure may be a protein having the activity of GcvP, GcvT, and GcvH proteins encoded by the *gcvP*, *gcvT*, and *gcvH* genes, respectively, but the type is not particularly limited as long as it has an activity corresponding to the glycine degradation enzyme. The GcvP, GcvT, and GcvH proteins encoded by the *gcvP*, *gcvT*, and *gcvH* genes, respectively, are known in the art, and the amino acid and polynucleotide sequences of each GcvP, GcvT, and GcvH protein can be obtained from a known database, NCBI's GenBank, *etc.,* but is not limited thereto.

In one example, the GcvP, GcvT, and GcvH proteins of the present disclosure may include amino acid sequences of SEQ ID NO: 47 SEQ ID NO: 49, and SEQ ID NO: 51, respectively, or an amino acid sequence with at least 60% homology or identity thereto, but is not limited thereto as long as it has an activity of the glycine degradation enzyme. Specifically, polypeptides having the activity of GcvP, GcvT, and GcvH proteins may have, include, consist of, or essentially consist of the amino acid sequences of SEQ ID NO: 47 SEQ ID NO: 49, and SEQ ID NO: 51, respectively, or amino acid sequences having at least 60%, 70%, 80%, 85%, 90%, 95%, 96%, 97%, 98%, or 99% or more homology or identity to SEQ ID NO: 47 SEQ ID NO: 49, and SEQ ID NO: 51, respectively. In one example, the GcvP, GcvT, and GcvH proteins may refer to proteins endogenously present in microorganisms of the genus *Corynebacterium* or *Corynebacterium stationis,* but are not limited thereto. Specifically, the GcvP, GcvT, and GcvH proteins may be GcvP protein consisting of the amino acid sequence of SEQ ID NO: 47, GcvT protein consisting of the amino acid sequence of SEQ ID NO: 49, and GcvH protein consisting of the amino acid sequence of SEQ ID NO: 51, respectively, which are endogenously present in microorganisms of the genus *Corynebacterium* or *Corynebacterium stationis,* but are not limited thereto.

Additionally, the nucleotide sequences encoding the GcvP, GcvT, and GcvH proteins may be a nucleotide sequence encoding a protein showing the activity of glycine degradation enzyme.

For example, the GcvP, GcvT, and GcvH proteins having the amino acid sequences of SEQ ID NO: SEQ ID NO: 47 SEQ ID NO: 49, and SEQ ID NO: 51, respectively, may be encoded by a polynucleotide that may have, include, consist of, essentially consist of nucleotide sequences of SEQ ID NO: 48, SEQ ID NO: 50, and SEQ ID NO: 52, respectively, or nucleotide sequences having 60% or more, 70% or more, 75% or more, 80% or more, 85% or more, 90% or more, 95% or more, 96% or more, 97% or more, 98% or more, and less than 100% homology or identity to the nucleotide sequences of SEQ ID NO: 48, SEQ ID NO: 50, and SEQ ID NO: 52, respectively, but are not limited thereto. In addition, the nucleotide sequences of SEQ ID NO: 48, SEQ ID NO: 50, and SEQ ID NO: 52 can be obtained from a known database, NCBI's GenBank, *etc.,* but is not limited thereto.

In one example, the microorganism having a purine nucleotide producing ability of the present disclosure may be a microorganism with an enhanced purine nucleotide producing ability by further enhancing the activity of serine hydroxymethyltransferase compared to the endogenous activity thereof, but is not limited thereto.

As used herein, the "serine hydroxymethyltransferase" is an enzyme that catalyzes the conversion of serine to glycine. The serine hydroxymethyltransferase of the present disclosure may be used interchangeably with GlyA. Specifically, the serine hydroxymethyltransferase of the present disclosure may be a protein having the activity of serine hydroxymethyltransferase encoded by the *glyA* gene, but the type thereof is not particularly limited as long as the protein has an activity corresponding to serine hydroxymethyltransferase. The serine hydroxymethyltransferase encoded by the *glyA* gene is known in the art, and the amino acid and polynucleotide sequences of the serine hydroxymethyltransferase can be obtained from a known database, NCBI's GenBank. *etc.*, but is not limited thereto.

In one example, the serine hydroxymethyltransferase may include an amino acid sequence of SEQ ID NO: 53 or an amino acid sequence having 60% or more homology thereto, but is not limited thereto as long as it has an activity of serine hydroxymethyltransferase. Specifically, a polypeptide having the activity of serine hydroxymethyltransferase may have, include, consist of, or essentially consist of the amino acid sequence of SEQ ID NO: 53, or an amino acid sequence having at least 60%, 70%, 80%, 85%, 90%, 95%, 96%, 97%, 98%, or 99% or more homology or identity to SEQ ID NO: 53. In one example, the serine hydroxymethyltransferase may refer to a protein endogenously present in microorganisms of the genus *Corynebacterium* or *Corynebacterium stationis,* but is not limited thereto. Specifically, it may be serine hydroxymethyltransferase consisting of the amino acid sequence of SEQ ID NO: 53, which is endogenously present in microorganisms of the genus *Corynebacterium* or *Corynebacterium stationis,* but is not limited thereto.

Additionally, the nucleotide sequence encoding the serine hydroxymethyltransferase may be a nucleotide sequence encoding a protein showing the activity of serine hydroxymethyltransferase.

For example, the serine hydroxymethyltransferase having the amino acid sequence of SEQ ID NO: 53 may be encoded by a polynucleotide that may have, include, consist of, essentially consist of a nucleotide sequence of SEQ ID NO: 54, or a nucleotide sequence having 60% or more, 70% or more, 75% or more, 80% or more, 85% or more, 90% or more, 95% or more, 96% or more, 97% or more, 98% or more, and less than 100% homology or identity to the sequence of SEQ ID NO: 54, but is not limited thereto. In addition, the nucleotide sequence of SEQ ID NO: 54 can be obtained from a known database, NCBI's GenBank, *etc.,* but is not limited thereto.

In one example, the microorganism having a purine nucleotide producing ability of the present disclosure may be a microorganism with an enhanced purine nucleotide producing ability by further enhancing the activity of formate-dependent phosphoribosylglycinamide formyltransferase compared to the endogenous activity thereof, but is not limited thereto

As used herein, the term "formate-dependent phosphoribosylglycinamide formyltransferase" has the activity of catalyzing the following chemical reaction:

10-formyltetrahydrofolate + *N*1-(5-phospho-D-ribosyl)glycinamide ⇔ tetrahydrofolate + N2-formyl-*N*1-(5-phospho-D-ribosyl)glycinamide

The formate-dependent phosphoribosylglycinamide formyltransferase of the present disclosure may be used interchangeably with PurT. Specifically, the formate-dependent phosphoribosylglycinamide formyltransferase of the present disclosure may be a protein having the activity of formate-dependent phosphoribosylglycinamide formyltransferase encoded by the *purT* gene, but the type thereof is not particularly limited as long as the protein has an activity corresponding to formate-dependent phosphoribosylglycinamide formyltransferase. The formate-dependent phosphoribosylglycinamide formyltransferase encoded by the *purT* gene is known in the art, and the amino acid and polynucleotide sequences of the formate-dependent phosphoribosylglycinamide formyltransferase can be obtained from a known database, NCBI's GenBank. *etc.*, but is not limited thereto.

In one example, the formate-dependent phosphoribosylglycinamide formyltransferase may include an amino acid sequence of SEQ ID NO: 55 or an amino acid sequence having 60% or more homology thereto, but is not limited thereto as long as it has an activity of formate-dependent phosphoribosylglycinamide formyltransferase. Specifically, a polypeptide having the activity of formate-dependent phosphoribosylglycinamide formyltransferase may have, include, consist of, or essentially consist of the amino acid sequence of SEQ ID NO: 55, or an amino acid sequence having at least 60%, 70%, 80%, 85%, 90%, 95%, 96%, 97%, 98%, or 99% or more homology or identity to SEQ ID NO: 55. In one example, the formate-dependent phosphoribosylglycinamide formyltransferase may refer to a protein endogenously present in microorganisms of the genus *Corynebacterium* or *Corynebacterium stationis,* but is not limited thereto. Specifically, it may be formate-dependent phosphoribosylglycinamide formyltransferase consisting of the amino acid sequence of SEQ ID NO: 55, which is endogenously present in microorganisms of the genus *Corynebacterium* or *Corynebacterium stationis,* but is not limited thereto.

Additionally, the nucleotide sequence encoding the formate-dependent phosphoribosylglycinamide formyltransferase may be a nucleotide sequence encoding a protein showing the activity of formate-dependent phosphoribosylglycinamide formyltransferase.

For example, the formate-dependent phosphoribosylglycinamide formyltransferase having the amino acid sequence of SEQ ID NO: 55 may be encoded by a polynucleotide that may have, include, consist of, essentially consist of a nucleotide sequence of SEQ ID NO: 56, or a nucleotide sequence having 60% or more, 70% or more, 75% or more, 80% or more, 85% or more, 90% or more, 95% or more, 96% or more, 97% or more, 98% or more, and less than 100% homology or identity to the sequence of SEQ ID NO: 56, but is not limited thereto. In addition, the nucleotide sequence of SEQ ID NO: 56 can be obtained from a known database, NCBI's GenBank, *etc.,* but is not limited thereto.

In one example, the microorganism having a purine nucleotide producing ability of the present disclosure may be a microorganism with an enhanced purine nucleotide producing ability by further weakening the activity of serine dehydratase compared to the endogenous activity thereof, but is not limited thereto.

As used herein, the "serine dehydratase (L-serine dehydratase)" is an enzyme that catalyzes the chemical reaction of L-serine ↔ pyruvate + NH₃. The serine dehydratase of the present disclosure may be used interchangeably with SdaA or L-serine ammonia-lyase. Specifically, the serine dehydratase of the present disclosure may be a protein having the activity of serine dehydratase encoded by the *sdaA* gene, but the type thereof is not particularly limited as long as the protein has an activity corresponding to serine dehydratase. The serine dehydratase encoded by the *sdaA* gene is known in the art, and the amino acid and polynucleotide sequences of the serine dehydratase can be obtained from a known database, NCBI's GenBank. *etc.*, but is not limited thereto.

In one example, the serine dehydratase may include an amino acid sequence of SEQ ID NO: 45 or an amino acid sequence having 60% or more homology thereto, but is not limited thereto as long as it has an activity of serine dehydratase. Specifically, a polypeptide having the activity of serine dehydratase may have, include, consist of, or essentially consist of the amino acid sequence of SEQ ID NO: 45, or an amino acid sequence having at least 60%, 70%, 80%, 85%, 90%, 95%, 96%, 97%, 98%, or 99% or more homology or identity to SEQ ID NO: 45. In one example, the serine dehydratase may refer to a protein endogenously present in microorganisms of the genus *Corynebacterium* or *Corynebacterium stationis,* but is not limited thereto. Specifically, it may be serine dehydratase consisting of the amino acid sequence of SEQ ID NO: 45, which is endogenously present in microorganisms of the genus *Corynebacterium* or *Corynebacterium stationis,* but is not limited thereto.

Additionally, the nucleotide sequence encoding the serine dehydratase may be a nucleotide sequence encoding a protein showing the activity of serine dehydratase.

For example, the serine dehydratase having the amino acid sequence of SEQ ID NO: 45 may be encoded by a polynucleotide that may have, include, consist of, essentially consist of a nucleotide sequence of SEQ ID NO: 46, or a nucleotide sequence having 60% or more, 70% or more, 75% or more, 80% or more, 85% or more, 90% or more, 95% or more, 96% or more, 97% or more, 98% or more, and less than 100% homology or identity to the sequence of SEQ ID NO: 46, but is not limited thereto. In addition, the nucleotide sequence of SEQ ID NO: 46 can be obtained from a known database, NCBI's GenBank, *etc.,* but is not limited thereto.

In the microorganism according to any one of the above-described embodiments, the microorganism having a purine nucleotide producing ability of the present disclosure may be a microorganism with an enhanced purine nucleotide producing ability by weakening the activity of formate dehydrogenase compared to the endogenous activity thereof, and enhancing the activity of formate-dependent phosphoribosylglycinamide formyltransferase compared to the endogenous activity thereof, but is not limited thereto.

In the microorganism according to any one of the above-described embodiments, the microorganism having a purine nucleotide producing ability of the present disclosure may be a microorganism with an enhanced purine nucleotide producing ability by weakening the activity of formate dehydrogenase compared to the endogenous activity thereof, enhancing the activity of formate-dependent phosphoribosylglycinamide formyltransferase compared to the endogenous activity thereof, and enhancing the activity of glycine degradation enzyme compared to the endogenous activity thereof, but is not limited thereto.

In the microorganism according to any one of the above-described embodiments, the microorganism having a purine nucleotide producing ability of the present disclosure may be a microorganism with an enhanced purine nucleotide producing ability by weakening the activity of serine dehydratase compared to the endogenous activity thereof, enhancing the activity of serine hydroxymethyltransferase compared to the endogenous activity thereof, enhancing the activity of glycine degradation enzyme compared to the endogenous activity thereof, weakening the activity of formate dehydrogenase compared to the endogenous activity thereof, and enhancing the activity of formate-dependent phosphoribosylglycinamide formyltransferase compared to the endogenous activity thereof, but is not limited thereto.

Another aspect of the present disclosure provides a method for producing purine nucleotides, including: culturing the microorganism of the present disclosure, in which the activity of formate dehydrogenase is weakened compared to the endogenous activity thereof, in a medium.

As used herein, the term "cultivation" means that the strain of the present disclosure is grown under appropriately controlled environmental conditions. The cultivation process of the present disclosure may be performed in a suitable culture medium and culture conditions known in the art. Such a cultivation process may be easily adjusted for use by those skilled in the art according to the strain to be selected. Specifically, the cultivation may be a batch culture, a continuous culture, and/or a fed-batch culture, but is not limited thereto.

As used herein, the term "medium" refers to a mixture of materials which contains nutrient materials required for the cultivation of the microorganism of the present disclosure as a main ingredient, and it supplies nutrient materials and growth factors, along with water that is essential for survival and growth. Specifically, the medium and other culture conditions used for culturing the strain of the present disclosure may be any medium used for conventional cultivation of microorganisms without any particular limitation. However, the microorganism of the present disclosure may be cultured under aerobic conditions in a conventional medium containing an appropriate carbon source, nitrogen source, phosphorus source, inorganic compound, amino acid, and/or vitamin, while adjusting temperature, pH, *etc.* For example, the culture medium for the microorganisms of the genus *Corynebacterium* can be found in the literature ("Manual of Methods for General Bacteriology" by the American Society for Bacteriology (Washington D.C., USA, 1981)).

In the present disclosure, the carbon source may include carbohydrates, such as glucose, saccharose, lactose, fructose, sucrose, maltose, *etc.;* sugar alcohols, such as mannitol, sorbitol, *etc.;* organic acids, such as pyruvic acid, lactic acid, citric acid, *etc.;* amino acids, such as glutamic acid, methionine, lysine, *etc.* Additionally, the carbon source may include natural organic nutrients such as starch hydrolysate, molasses, blackstrap molasses, rice bran, cassava, sugar cane molasses, and corn steep liquor, *etc.* Specifically, carbohydrates such as glucose and sterilized pretreated molasses (*i.e*., molasses converted to reducing sugar) may be used, and in addition, various other carbon sources in an appropriate amount may be used without limitation. These carbon sources may be used alone or in a combination of two or more kinds, but are not limited thereto.

The nitrogen source may include inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc.;* and organic nitrogen sources, such as peptone, NZ-amine, meat extract, yeast extract, malt extract, corn steep liquor, casein hydrolysate, fish or decomposition product thereof, defatted soybean cake or decomposition product thereof, *etc.* These nitrogen sources may be used alone or in a combination of two or more kinds, but are not limited thereto.

The phosphorus source may include monopotassium phosphate, dipotassium phosphate, or corresponding sodium-containing salts, *etc.* Examples of the inorganic compounds may include sodium chloride, calcium chloride, iron chloride, magnesium sulfate, iron sulfate, manganese sulfate, calcium carbonate, *etc.* Additionally, amino acids, vitamins, and/or appropriate precursors, *etc.,* may be included. These constituting ingredients or precursors may be added to a medium in a batch or continuous manner, but these phosphorus sources are not limited thereto.

Additionally, the pH of the medium may be adjusted by adding a compound such as ammonium hydroxide, potassium hydroxide, ammonia, phosphoric acid, sulfuric acid, *etc.* during the cultivation of the microorganism of the present disclosure in an appropriate manner. In addition, bubble formation may be prevented during the cultivation using an antifoaming agent such as fatty acid polyglycol ester. Further, oxygen gas or a gas containing oxygen may be injected to the medium order to maintain aerobic conditions of the medium; or nitrogen gas, hydrogen gas, or carbon dioxide may be injected to maintain anaerobic or microaerobic conditions, without the injection of gas, but the gas is not limited thereto.

The temperature during the cultivation of the present disclosure may be in the range from 27°C to 37°C, specifically from 30°C to 33°C, and the cultivation may be continued for 20 hours to 120 hours, but is not limited thereto.

As used herein, the term "culture product" means a culture solution, a concentrated culture solution, a dried product of a culture solution, a culture filtrate, a concentrated culture filtrate, or a dried product of a culture filtrate obtained by culturing a specific microorganism in a culture medium, and means that the culture solution may include the specific microorganism while the culture filtrate does not substantially include the specific microorganism (in particular, it substantially means excluding a specific microorganism isolated by filtration, *etc.,* but does not mean that the microorganism is completely excluded from the filtrate). The formulation of the culture product is not limited, and may be, for example, a liquid, emulsion, or solid. Specifically, for the purpose of the present disclosure, the culture product may include purine nucleotides.

As used herein, the term "fermentation" means that microorganisms are not putrefactive during the process of decomposing organic matter using their own enzymes. Fermentation reaction and decay reaction proceed by similar processes, but when decomposition produces useful substances, it is called fermentation, and when odorous or harmful substances are produced, it is called decay.

In the present disclosure, the method for obtaining a fermented product from the strain is not particularly limited, and may be obtained according to a method commonly used in the art or similar fields.

As used herein, the term "fermented product" may include not only the fermented material itself, but also all kinds of materials including fermented products produced from the strain, such as a culture medium of the strain in which the strain and the culture coexist, a fermented product produced from the culture medium, a fermented product obtained by filtering the strain from the culture medium, a fermented product obtained by sterilizing the strain from the culture medium and filtering the same, an extract obtained by extracting the fermented product or a culture medium containing the same, a diluted solution obtained by diluting the fermented product or an extract thereof, a concentrated solution, a dried product obtained by drying the fermented product or an extract thereof, and a lysate obtained by collecting and lysing the cells of the strain, *etc.*

In the method of the present disclosure, the cultivation of the microorganism may be performed by any culture conditions and culture methods known in the art. Such a cultivation process can be easily adjusted and used by those skilled in the art according to the selected strain.

The purine nucleotides produced by the cultivation of the present disclosure may be released into the medium or remain in the cells.

In one embodiment, the method for producing purine nucleotides of the present disclosure may further include a step of preparing the microorganism of the present disclosure, a step of preparing a medium for culturing the strain, or a combination thereof (regardless of the order, in any order), for example, prior to the culturing step.

The method for producing purine nucleotides of the present disclosure may further include recovering the target substances, specifically, purine nucleotides, from the cultured microorganism, a culture product of the microorganism, a fermented product of the microorganism, or the culture medium. The recovering step may be further included after the culturing step.

In the recovering step, the target purine nucleotides may be collected using the method of culturing the microorganism of the present disclosure, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatography such as molecular sieve chromatography (gel filtration), adsorption chromatography, ion exchange chromatography, affinity chromatography, *etc.,* HPLC or a combination thereof may be used, and the target substances, specifically, purine nucleotides, can be recovered from the medium or the microorganisms using suitable methods known in the art.

Further, the method for producing purine nucleotides of the present disclosure may further include a purification step, which may be performed using an appropriate method known in the art. In one example, when the method for producing purine nucleotides of the present disclosure includes both a recovering step and a purification step, the recovering step and the purification step may be performed continuously or intermittently regardless of the order or simultaneously, or may be integrated into one step, but the method is not limited thereto.

In the method of the present disclosure, the weakening of the activity of formate dehydrogenase and purine nucleotides, *etc.* are as described in other aspects above.

Still another aspect of the present disclosure provides a composition for producing purine nucleotides: including the microorganism of the present disclosure, in which the activity of formate dehydrogenase is weakened compared to the endogenous activity thereof, a culture product of the microorganism, a fermented product of the microorganism, or a combination of two or more thereof.

The composition of the present disclosure may further include any suitable excipient commonly used in compositions for producing purine nucleotides, and such excipients may include, for example, preservatives, wetting agents, dispersing agents, suspending agents, buffers, stabilizers, or isotonic agents, *etc.,* but are not limited thereto.

In one embodiment, each component present in the composition of the present disclosure may be contained in a microbiologically effective amount, or in an amount that can be appropriately present in the composition for production.

In the composition of the present disclosure, the weakening of the activity of formate dehydrogenase and purine nucleotides, *etc.* are as described in other aspects above.

Yet another aspect of the present disclosure provides the use of the microorganism of the present disclosure, in which the activity of formate dehydrogenase is weakened compared to the endogenous activity thereof, for the production of purine nucleotides.

In the use of the present disclosure, the weakening of the activity of formate dehydrogenase and purine nucleotides, *etc.* are as described in other aspects above.

### [Mode for Carrying Out the Invention]

Hereinafter, the present disclosure will be described in detail by way of Examples. However, these Examples are merely preferred Examples given for illustrative purposes, and thus, the scope of the present disclosure is not intended to be limited to or by these Examples. Meanwhile, technical features which are not described herein can be sufficiently understood and easily carried out by those skilled in the art in the technical field of the present disclosure or in a similar technical field.

### Example 1. Preparation of Formate Dehydrogenase-Deleted Strain, Preparation of Formate-Dependent Phosphoribosylglycinamide Formyltransferase-Enhanced Strain, and Evaluation of IMP Production Ability

### Example 1-1. Preparation of Formate Dehydrogenase-Deleted Recombinant Vector and IMP-Producing Strain

*Corynebacterium stationis*-derived formate dehydrogenase may consist of formate dehydrogenase subunit 1 (Fdh subunit 1), formate dehydrogenase subunit 2 (Fdh subunit 2), and formate dehydrogenase subunit 3 (Fdh subunit 3).

An experiment was conducted to prepare a strain, in which the endogenous formate dehydrogenase was deleted from *Corynebacterium stationis* KCCM12151P, an IMP-producing strain, to confirm whether the increase of formate by deletion of the formate dehydrogenase subunit 1, formate dehydrogenase subunit 2, and formate dehydrogenase subunit 3 (hereinafter referred to as formate dehydrogenase) could lead to an increase in IMP production.

Specifically, an experiment was conducted to prepare a strain in which the *fdh 1, fdh 2,* and *fdh* 3 genes (hereinafter referred to as *fdh* gene) encoding the formate dehydrogenase subunit 1, formate dehydrogenase subunit 2, and formate dehydrogenase subunit 3, respectively, were deleted.

The chromosomal gene of ATCC6872 strain, which is a wild-type *Corynebacterium stationis,* was isolated using a G-spin total DNA extraction mini kit (Cat. No. 17045, Intron) according to a protocol provided in the kit, and PCR was performed using a primer pair of SEQ ID NOS: 1 and 2 and a primer pair of SEQ ID NOS: 3 and 4 to obtain a pair of gene fragments (fdh-A, fdh-B), respectively. PCR was performed under conditions of denaturation at 94°C for 5 minutes, followed by 20 cycles of denaturation at 94°C for 30 seconds, annealing at 55°C for 30 seconds, and polymerization at 72°C for 1 minute, and then polymerization at 72°C for 7 minutes. As a result, polynucleotides of 1091 bp and 1105 bp were obtained, respectively.

A polynucleotide template of 2.2 kbp was obtained by performing secondary PCR using the two fragments as a template. The obtained gene fragments were cleaved with restriction enzyme Xbal (New England Biolabs, Beverly, MA). The gene fragments, which were cleaved with Xbal restriction enzyme, were ligated to linear pDCM2 (Korean Patent Publication No. 10-2020-0136813) using T4 ligase (New England Biolabs, Beverly, MA). The resulting vector was named pDCM2-del_fdh.

The sequences of the primers used for vector preparation are as follows

**[Table 1]**

| SEQ ID NO: | Name | Sequence |
|---|---|---|
| 1 | fdh-A_F | 5′- AATTCGAGCTCGGTACCCGTTGCCGTATCA GACATGCT-3′ |
| 2 | fdh-A-R | 5′- TCATAGGTGCCGAACTTGATTTATCTCGACC AAAC-3′ |
| 3 | fdh-B-F | 5′- GTCGAGATAAATCAAGTTCGGCACCTATGAG AATA-3′ |
| 4 | fdh-B-R | 5′- CGACTCTAGAGGATCCCCCTCGGGTTCCAT GTTGTCTT-3′ |

The pDCM2-del_fdh vector was transformed into the *Corynebacterium stationis* KCCM12151P (IMP-producing strain), which is a strain having IMP producing ability, by electroporation, and then the strain inserted with the mutant gene and the vector on the chromosomes was selected as primary candidates in a selection medium containing 25 mg/L kanamycin. Thereafter, an endogenous formate dehydrogenase on the chromosome was deleted through a secondary crossover process using homology between the gene existing on the chromosome and the gene inserted through the vector, and a final strain, from which the vector containing a kanamycin resistance gene was removed, was obtained. The strain with deletion was primarily confirmed through PCR using primers of SEQ ID NOS: 5 and 6, and was finally confirmed through gene sequencing analysis. The strain in which the formate dehydrogenase was deleted, obtained by the method above was named CJI-3162(KCCM12151 P_fdh_deletion) and was deposited at the Korean Culture Center of Microorganisms (KCCM), a Depositary Authority, under the Budapest Treaty on November 24, 2022 with Accession No. KCCM13275P.

**[Table 2]**

| SEQ ID NO: | Name | Sequence |
|---|---|---|
| 5 | fdh-deletion-confirm_F | 5'-CTTGTGCGCCGAAGCCCGTT-3' |
| 6 | fdh-deletion-confirm_R | 5'-GCTTTCATGAGCTTGCCGGG-3' |

### Example 1-2. Preparation of Integrated Strain with Deletion of Formate Dehydrogenase and Enhancement of Formate-Dependent Phosphoribosylglycinamide Formyltransferase and Preparation of IMP-Producing Strain

A strain integrated with the deletion of formate dehydrogenase and enhancement of formate-dependent phosphoribosylglycinamide formyltransferase was prepared and evaluated.

In order to prepare a strain, in which an endogenous *purT* gene activity was additionally enhanced in the CJI-3162 strain produced in Example 1-1, an experiment was conducted to enhance *purT* in the form of Pcj7 promoter (Korean Application No. 10-2004-0107215).

The chromosomal gene of ATCC6872 strain, which is a wild-type *Corynebacterium stationis,* was isolated using a G-spin total DNA extraction mini kit (Cat. No. 17045, Intron) according to a protocol provided in the kit, and PCR was performed using a primer pair of SEQ ID NOS: 7 and 8, a primer pair of SEQ ID NOS: 9 and 10, and a primer pair of SEQ ID NOS: 11 and 12 to obtain a pair of gene fragments (Pcj7/purT-A, Pcj7/purT-B, Pcj7/purT-C), respectively. PCR was performed under conditions of denaturation at 94°C for 5 minutes, followed by 20 cycles of denaturation at 94°C for 30 seconds, annealing at 55°C for 30 seconds, and polymerization at 72°C for 1 minute, and then polymerization at 72°C for 7 minutes. As a result, polynucleotides of 851 bp, 318 bp, and 882 bp were obtained, respectively.

A polynucleotide template of 2 kbp was obtained by performing secondary PCR using the three fragments as a template. The obtained gene fragments were cleaved with restriction enzyme Xbal (New England Biolabs, Beverly, MA). The gene fragments, which were cleaved with Xbal restriction enzyme, were ligated to linear pDCM2 (Korean Patent Publication No. 10-2020-0136813) using T4 ligase (New England Biolabs, Beverly, MA). The resulting vector was named pDCM2-Pcj7/purT.

The sequences of primers used for vector preparation are as follows.

**[Table 3]**

| SEQ ID NO: | Name | Sequence |
|---|---|---|
| 7 | Pcj7/purT-A-F | 5′- AATTCGAGCTCGGTACCCAATCCATCCTGC GTCAGAAG-3′ |
| 8 | Pcj7/purT-A-R | 5′- cgctgggatgtttctGGTTGCTCCTTTTTATTATT-3′ |
| 9 | Pcj7/purT-B-F | 5′- TAAAAAGGAGCAACCagaaacatcccagcgctact- 3′ |
| 10 | Pcj7/purT-B-R | 5'-GATATAAGACTCCAAgagtgtttcctttcgttggg-3' |
| 11 | Pcj7/purT-C-F | 5′- cgaaaggaaacactcTTGGAGTCTTATATCGGTA G-3′ |
| 12 | Pcj7/purT-C-R | 5′- CGACTCTAGAGGATCCCCCATCGCTTTATC AGAAGGAC-3′ |

The pDCM2-Pcj7/purT vector was transformed into the CJI-3162 (KCCM12151P_fdh_deletion) prepared in Example 1-1 by electroporation, and then the strain inserted with the mutant gene and the vector on the chromosomes was selected as primary candidates in a selection medium containing 25 mg/L kanamycin. Thereafter, an endogenous *purT* promoter was enhanced with Pcj7 through a secondary crossover process using homology between the gene existing on the chromosome and the gene inserted through the vector, and a final strain, from which the vector containing a kanamycin resistance gene was removed, was obtained. The *purT*-enhanced strain was primarily confirmed through PCR using primers of SEQ ID NOS: 13 and 14, and was finally confirmed through gene sequencing analysis. The Pcj7/purT strain obtained by the method above was named CJI-3163 (CJI3162_Pcj7/purT).

**[Table 4]**

| SEQ ID NO: | Name | Sequence |
|---|---|---|
| 13 | Pcj7/purT-confirm_F | 5'-CGAATACCTCCAGACCACCG-3' |
| 14 | Pcj7/purT-confirm_R | 5'-TGGGCACAACGGTGACAGCG-3' |

### Example 1-3. Evaluation of IMP Production Ability of Two Strains of Formate Dehydrogenase-Deleted Strain and Formate-Dependent Phosphoribosylglycinamide Formyltransferase-Enhanced Strain

The IMP production ability of the strains prepared above was evaluated through flask evaluation. The *Corynebacterium stationis* KCCM12151P, CJI-3162, and CJI-3163 were inoculated into a 14 mL tube containing 2.5 mL of the following seed medium, and incubated at 30°C and 170 rpm for 24 hours with shaking. Then, 2 mL of the seed culture solution was inoculated into a 250 mL corner-baffle flask containing 29 mL of the following production medium (24 mL of main medium + 5 mL of separate sterile medium), and incubated at 30°C and 170 rpm for 72 hours with shaking. After completion of the culture, the IMP production ability was measured by HPLC.

The compositions of the seed medium and the fermentation medium are as follows.

### <IMP Seed Medium>

glucose 1%, peptone 1%, meat extract 1%, yeast extract 1%, NaCl 0.25%, adenine 100 mg/L, guanine 100 mg/L, pH 7.2

### <IMP Flask Fermentation Medium>

Sodium glutamate 0.1%, ammonium chloride 1%, magnesium sulfate 1.2%, calcium chloride 0.01%, iron sulfate 20 mg/L, manganese sulfate 20 mg/L, iron sulfate 20 mg/L, copper sulfate 5 mg/L, L-cystine 23 mg/L, beta-alanine 24 mg/L, nicotinic acid 8 mg/L, biotin 45 µg/L, thiamine-HCl 5 mg/L, adenine 30 mg/L, phosphoric acid (85%) 1.9%, glucose 4.2%, fructose 2.4% added

The results of the culture according to the deletion of formate dehydrogenase and enhancement of formate-dependent phosphoribosylglycinamide formyltransferase of *Corynebacterium stationis* are shown in Table 5 below.

**[Table 5]**

| Confirmation of IMP Production according to Deletion of Formate Dehydrogenase and Enhancement of Formate-Dependent Phosphoribosylglycinamide Formyltransferase | | | | |
|---|---|---|---|---|
| Strain No. | Type of Introduction | OD | IMP(g/L) | Concentration Increase Rate (%) |
| KCCM12151P | Control | 40.3 | 5.4 | - |
| CJI-3162 | Δfdh | 41.3 | 5.8 | 7.4 |
| CJI-3163 | CJI03162-Pcj7/purT | 40.8 | 6.1 | 12.9 |

The fdh-deleted strain in the IMP-producing strain showed an increased IMP production ability by 7.4% compared to the control group, and it was confirmed that the IMP production ability was further enhanced when the *purT* gene was enhanced in the fdh-deleted strain.

### Example 2. Preparation and Evaluation of Strain with gcvPTH Enhancement, sdaA Deletion, and glyA Enhancement based on Strain with Deletion of Formate Dehydrogenase and Enhancement of Formate-Dependent Phosphoribosylglycinamide Formyltransferase

### Example 2-1. Preparation of Recombinant Vector Enhaced by gcvPTH Gene Addition

A copy of *gcvPTH* was added.

A vector which adds a copy of the endogenous *gcvPTH* gene of the *Corynebacterium stationis* strain in the form of a native promoter was prepared as follows using the plasmid pDCM2 (Korea Patent Publication No. 10-2020-0136813) for insertion and replacement of genes in the chromosomes of *Corynebacterium.*

The chromosomal gene of ATCC6872 strain, which is a wild-type *Corynebacterium stationis,* was isolated using a G-spin total DNA extraction mini kit (Cat. No. 17045, Intron) according to a protocol provided in the kit, and PCR was performed using a primer pair of SEQ ID NOS: 15 and 16, a primer pair of SEQ ID NOS: 17 and 18, a primer pair of SEQ ID NOS: 19 and 20, and a primer pair of SEQ ID NOS: 21 and 22 to obtain gene fragments (Pn/gcvPTH-A, Pn/gcvPTH-B, Pn/gcvPTH -C, Pn/gcvPTH-D, respectively. PCR was performed under conditions of denaturation at 94°C for 5 minutes, followed by 20 cycles of denaturation at 94°C for 30 seconds, annealing at 55°C for 30 seconds, and polymerization at 72°C for 1 minute, and then polymerization at 72°C for 7 minutes.

The obtained gene fragments were cleaved with restriction enzyme Xbal (New England Biolabs, Beverly, MA). The gene fragments, which were cleaved with Xbal restriction enzyme, were ligated to linear pDCM2 (Korean Patent Publication No. 10-2020-0136813) using T4 ligase (New England Biolabs, Beverly, MA). The resulting vector was named pDCM2-Pn/gcvPTH.

The sequences of primers used for vector preparation are as follows.

**[Table 6]**

| SEQ ID NO: | Name | Sequence |
|---|---|---|
| 15 | Pn/gcvPTH-A-F | 5′- AATTCGAGCTCGGTACCCAGGCACAGCCG ACATTACGG-3′ |
| 16 | Pn/gcvPTH-A-R | 5′- GTAGCACATCTGACGAATGTCCAAGTCTAA GGAAA-3′ |
| 17 | Pn/gcvPTH-B-F | 5′- TTAGACTTGGACATTCGTCAGATGTGCTAC TTGCC-3′ |
| 18 | Pn/gcvPTH-B-R | 5′- TGCGCCATTAGCGTGTTACTTCTCGCGGCT ATAGA-3′ |
| 19 | Pn/gcvPTH-C-F | 5′- AGCCGCGAGAAGTAACACGCTAATGGCGC ATTGAA-3′ |
| 20 | Pn/gcvPTH-C-R | 5′- TGAATTATCCGCGTCTTAGATGCCGTTTTC TGCCG-3′ |
| 21 | Pn/gcvPTH-D-F | 5′- GAAAACGGCATCTAAGACGCGGATAATTCA GCTGT-3′ |
| 22 | Pn/gcvPTH-D-R | 5′- CGACTCTAGAGGATCCCCGATGAAGCGAA CACTTAAAT-3′ |

### Example 2-2. Preparation of IMP-Producing Strain in which gcvPTH Gene is Additionally Enhanced

An experiment was conducted to further enhance *gcvPTH* in the CJI-3163 (CJI3162_Pcj7/purT) prepared in Example 1-2 above. The pDCM2-Pn/gcvPTH vector prepared in Example 2-1 was transformed into the CJI-3163 (CJI3162_Pcj7/purT) prepared in Example 1-2 by electroporation, and then the strain inserted with the mutant gene and the vector on the chromosomes was selected as primary candidates in a selection medium containing 25 mg/L kanamycin. Thereafter, the strain was primarily confirmed through PCR using a primer pair of SEQ ID NOS: 23 and 24 in the strains, in which homologous recombination occurred, and was finally confirmed through gene sequencing analysis. The selected strain was named CJI-3188(CJI-3163_Pn/gcvPTH).

**[Table 7]**

| SEQ ID NO: | Name | Sequence |
|---|---|---|
| 23 | primer | 5'-CCAGTACATTGTCATGGGAT-3' |
| 24 | primer | 5'-GATTGGCATATCGCACCGAG-3' |

### Example 2-3. Preparation of Recombinant Vector Enhanced by sdaA Deletion and glyA Addition

A vector was prepared in the form of deleting *sdaA*, an endogenous gene of *Corynebacterium stationis* and adding a copy number of the *glyA* gene at the corresponding position.

The chromosomal gene of ATCC6872 strain, which is a wild-type *Corynebacterium stationis,* was isolated using a G-spin total DNA extraction mini kit (Cat. No. 17045, Intron) according to a protocol provided in the kit, and PCR was performed using a primer pair of SEQ ID NOS: 25 and 26, a primer pair of SEQ ID NOS: 27 and 28, a primer pair of SEQ ID NOS: 29 and 30, and a primer pair of SEQ ID NOS: 31 and 32 to obtain gene fragments (del_sda::Pcj7/glyA-A, del_sda::Pcj7/glyA-B, del_sda::Pcj7/glyA -C, del_sda::Pcj7/glyA-D), respectively. PCR was performed under conditions of denaturation at 94°C for 5 minutes, followed by 20 cycles of denaturation at 94°C for 30 seconds, annealing at 55°C for 30 seconds, and polymerization at 72°C for 1 minute, and then polymerization at 72°C for 7 minutes.

The gene fragments obtained by performing secondary PCR based on the four fragments obtained above were cleaved with restriction enzyme Xbal (New England Biolabs, Beverly, MA). The gene fragments, which were cleaved with Xbal restriction enzyme, were ligated to linear pDCM2 (Korean Patent Publication No. 10-2020-0136813) using T4 ligase (New England Biolabs, Beverly, MA). The resulting plasmid was named pDCM2-del_sdaA-Pn/glyA.

The sequences of the primers used for vector preparation are as follows.

**[Table 8]**

| SEQ ID NO: | Name | Sequence |
|---|---|---|
| 25 | del_sda::Pcj7/glyA -A_F | 5′- AATTCGAGCTCGGTACCCCATTTCGTGACC ATTAGCGT-3′ |
| 26 | del_sda::Pcj7/glyA -A_R | 5′- ATTAGCCTGAAGGAAGATCATGCCAATAGC GCCAG-3′ |
| 27 | del_sda::Pcj7/glyA -B_F | 5′- GCTATTGGCATGATCTTCCTTCAGGCTAAT CTTTT-3′ |
| 28 | del_sda::Pcj7/glyA-B_R | 5'- ATTCTGGGTAGTCATCATATGTGTTTCCTTT CGTT-3′ |
| 29 | del_sda::Pcj7/glyA -C_F | 5′- AAGGAAACACATATGATGACTACCCAGAAT TCTTC-3′ |
| 30 | del_sda::Pcj7/glyA -C_R | 5′- CTTTGTACTTGGTGACCAAGAGTCAGGATG CCGAA-3′ |
| 31 | del_sda::Pcj7/glyA -D_F | 5′- CATCCTGACTCTTGGTCACCAAGTACAAAG AGACT-3′ |
| 32 | del_sda::Pcj7/glyA -D_R | 5′- CGACTCTAGAGGATCCCCGGTTACCGCGC ATTAGGACT-3′ |

The pDCM2-del_sdaA-Pcj7/glyA vector was transformed into the CJI-3188 strain (CJI-3163_Pn/gcvPTH) prepared in Example 2-2 by electroporation, and then the strain inserted with the mutant gene and the vector on the chromosomes was selected as primary candidates in a selection medium containing 25 mg/L kanamycin. Thereafter, the strain, in which the *sdaA* was deleted and the *glyA* gene linked to Pcj7 promoter at the corresponding position was inserted, was selected using a primer pair of SEQ ID NOS: 34 and 35 in the strains, in which homologous recombination occurred. The strain obtained by the method above was named CJI-3189(CJI-3188_del _sdaA-Pn/glyA).

The sequences of primers used for strain preparation are as follows.

**[Table 9]**

| SEQ ID NO: | Name | Sequence |
|---|---|---|
| 33 | Primer | 5'-TAATCCCCCAGCTCACCGGC-3' |
| 34 | primer | 5'-TTTGGATGCCGAGGTCGTAG-3' |

### Example 2-4. Evaluation of IMP Production Ability of Combined Strain with gcvPTH Enhancement, sdaA Deletion, and glyA Enhancement based on Strain with Deletion of Formate Dehydrogenase and Enhancement of Formate-Dependent Phosphoribosylglycinamide Formyltransferase

Flask titer evaluation was performed to measure IMP production ability of the strains prepared in Example 2-3 and Example 2-4, and the parent strain. The *Corynebacterium stationis* KCCM12151P, CJI-3188, and CJI-3189 were inoculated into a 14 mL tube containing 2.5 mL of the following seed medium, and incubated at 30°C and 170 rpm for 24 hours with shaking. Then, 2 mL of the seed culture solution was inoculated into a 250 mL corner-baffle flask containing 29 mL of the following production medium (24 mL of main medium + 5 mL of separate sterile medium), and incubated at 30°C and 170 rpm for 72 hours with shaking. After completion of the culture, the IMP production ability was measured by HPLC.

The compositions of the seed medium and fermentation medium are the same as in Examples 1-3.

The results of the culture according to the combination of *gcvPTH* enhancement, *glyA* enhancement, and *sdaA* deletion with deletion of formate dehydrogenase and enhancement of formate-dependent phosphoribosylglycinamide formyltransferase of *Corynebacterium stationis* are shown in Table 10 below.

**[Table 10]**

| Confirmation of IMP Production according to Combination of *gcvPTH* Enhancement, *glyA* Enhancement, and *sdaA* Deletion with Deletion of formate dehydrogenase and Enhancement of formate-dependent phosphoribosylglycinamide formyltransferase | | | | |
|---|---|---|---|---|
| Strain No. | Type of Introduction | OD | IMP(g/L) | Concentrat ion Increase Rate (%) |
| KCCM12151P | Control | 40.3 | 5.4 | - |
| CJI-3188 | CJI-3163_Pn/gcvPTH | 39.2 | 6.3 | 16.7 |
| CJI-3189 | CJI-3188_delsda::Pcj7/glyA | 39.3 | 6.8 | 25.9 |

It was confirmed that the integration of *fdh* deletion, *purT* enhancement, and *gcvPTH* enhancement in the IMP-producing strain could lead to an increase in IMP production, and additionally the IMP production concentration was further enhanced when *sdaA* deletion and *glyA* enhancement were integrated.

### Example 3. Preparation of XMP (5'-Xanthosine Monophosphate)-Producing Strain with Deletion of Formate Dehydrogenase and Enhancement of Formate-Dependent Phosphoribosylglycinamide Formyltransferase and Evaluation of XMP Production Ability

### Example 3-1. Preparation of Formate Dehydrogenase-Deleted Recombinant Vector and XMP-Producing Strain

An experiment was conducted to prepare a strain, in which the endogenous formate dehydrogenase was deleted from an XMP-producing *Corynebacterium stationis* strain to confirm whether the increase of formate by deletion of the formate dehydrogenase could lead to an increase in XMP production. A vector for deleting the gene encoding formate dehydrogenase was prepared as follows using the plasmid pDCM2 (Korea Patent Publication No. 10-2020-0136813) for insertion and replacement of genes in the chromosomes of *Corynebacterium.*

PCR was performed based on the gDNA (genomic DNA) of the wild-type *Corynebacterium stationis* ATCC6872 as a template, using a primer pair of SEQ ID NOS: 35 and 36 and a primer pair of SEQ ID NOS: 37 and 38. PCR was performed under conditions of denaturation at 94°C for 5 minutes, followed by 30 cycles of denaturation at 94°C for 30 seconds, annealing at 55°C for 30 seconds, and polymerization at 72°C for 1 minute and 30 seconds, and then polymerization at 72°C for 5 minutes. The pDCM2 vector was treated with Xbal and the PCR product obtained above was subjected to fusion cloning. Fusion cloning was performed by the In-Fusion^{®} HD cloning kit (Clontech). The resulting plasmid was named pDCM2-del fdh.

The sequences of primers used for vector preparation are as follows.

**[Table 11]**

| SEQ ID NO: | Name | Sequence |
|---|---|---|
| 35 | primer | 5′- ACCCGGGGATCCTCTAGAGACATGCTCAG CACCTTTCTTC-3′ |
| 36 | primer | 5'-AGCCTTAAGGCTACCGGATC-3' |
| 37 | primer | 5′- TCCGGTAGCCTTAAGGCTATGCCGGATTTC GACGAGTATC-3′ |
| 38 | primer | 5′- AAGCTTGCATGCCTGCAGCGCCTTCAAGG ACGCCTAAG-3′ |

The vector prepared above was transformed into *Corynebacterium stationis* CJX1664 strain (KR 10-1950141 B1) by electroporation, and then the strain with the vector inserted into the chromosome was selected as primary candidates in a selection medium containing 25 mg/L kanamycin. Thereafter, the strain with deletion of the gene encoding formate dehydrogenase was selected using a primer pair of SEQ ID NOS: 35 and 38 in the strains, in which homologous recombination occurred. The selected strain was named CJX1664_del fdh.

### Example 3-2. Preparation of Formate-Dependent Phosphoribosylglycinamide Formyltransferase-Enhanced Recombinant Vector and XMP-Producing Strain

An experiment was conducted to prepare a strain, in which the expression of endogenous formate-dependent phosphoribosylglycinamide formyltransferase was enhanced in an XMP-producing *Corynebacterium stationis* strain to confirm whether the enhancement of formate-dependent phosphoribosylglycinamide formyltransferase could lead to an increase in XMP production ability.

At the same time, the expression of formate-dependent phosphoribosylglycinamide formyltransferase was enhanced in the formate dehydrogenase-deleted strain to confirm whether the increase of formate by deletion of formate dehydrogenase could lead to an additional increase in XMP production by formate-dependent phosphoribosylglycinamide formyltransferase.

The pDCM2-Pcj7/purT vector prepared in Example 1-2, which replaces the promoter of the *purT* gene encoding formate-dependent phosphoribosylglycinamide formyltransferase with a stronger promoter was transformed into each of *Corynebacterium stationis* CJX1664 strain (KR 10-1950141 B1) and CJX1664_del fdh, which is a formate dehydrogenase-deleted strain, prepared in Example 3-1, by electroporation, and then the strains with the vector inserted into the chromosome were selected as primary candidates in a selection medium containing 25 mg/L kanamycin. Thereafter, the strains, in which the *purT* gene was linked to Pcj promoter, were selected using a primer pair of SEQ ID NOS: 9 and 12 in the strains, in which homologous recombination occurred. The selected strains were named CJX1664_Pcj7/purT, CJX1664_del fdh_Pcj7/purT, respectively.

### Example 3-3. Evaluation of XMP Production Ability of Two Strains of Formate Dehydrogenase-Deleted Strain and Formate-Dependent Phosphoribosylglycinamide Formyltransferase-Enhanced Strain

The XMP production ability of the three strains prepared in Example 3-1 and Example 3-2 and the parent strain was evaluated through flask fermentation titer evaluation.

First, each strain was inoculated into an 18 mm diameter test tube containing 2 mL of the following seed medium, and incubated at 30°C for 24 hours with shaking, and used as seed culture solutions. Then, 0.7 mL of the seed culture solutions were inoculated into a 250 mL corner-baffle flask containing 32 mL of the following production medium (24 mL of main medium + 8 mL of separate sterile medium), and incubated at 30°C and 170 rpm for 75 hours with shaking. After completion of the culture, the XMP production ability was measured by HPLC. The concentration and concentration increase rate of XMP in the culture solution for each strain tested are shown in Table 3 below.

### <Seed Medium (pH7.5)>

glucose 1%, peptone 1%, meat extract 1%, yeast extract 1%, NaCl 0.25%, adenine 100 mg/L, guanine 100 mg/L (based on 1 L of distilled water)

### <XMP Flask Production Medium (Main Medium)>

glucose 40 g/L, magnesium sulfate 10 g/L, calcium chloride 100 mg/L, iron sulfate 20 mg/L, manganese sulfate 10 mg/L, zinc sulfate 10 mg/L, copper sulfate 0.8 mg/L, histidine 20 mg/L, cysteine 15 mg/L, beta-alanine 15 mg/L, biotin 100 µg/L, thiamine 5 mg/L, adenine 50 mg/L, guanine 25 mg/L, niacin 15 mg/L, pH 7.0

### <XMP Flask Production Medium (Separate Sterile Medium)>

potassium phosphate monobasic 18 g/L, potassium phosphate dibasic 42 g/L, urea 7 g/L, ammonium sulfate 5 g/L (based on 1 L of distilled water)

The above experiment was repeated three times, and the average values of the analysis results are shown in Table 12 below.

**[Table 12]**

| Comparison of XMP Production Ability According to Deletion of Formate Dehydrogenase and Enhancement of Formate-Dependent Phosphoribosylglycinamide Formyltransferase | | |
|---|---|---|
| Strain | XMP Concentration (g/L) | XMP Concentration Increase Rate (%) |
| CJX1664 | 4.27 | - |
| CJX1664_del fdh | 4.40 | 3.04% |
| CJX 1664_Pcj7/purT | 4.39 | 2.81% |
| CJX1664_del fdh-Pcj7/purT | 4.51 | 5.62% |

As shown in Table 12 above, the CJX1664_del fdh strain and the CJX1664_Pcj7/purT strain showed an increased XMP production ability by 3.04% and 2.81% compared to the control CJX1664, respectively, and the CJX1664_del fdh-Pcj7/purT showed an increased XMP production ability by 5.62%. Based on the results, it was confirmed that an increase in formate by deletion of formate dehydrogenase could can lead to an increase in XMP production, and that the XMP production ability was further increased when the formate-dependent phosphoribosylglycinamide formyltransferase, which utilizes the formate as a substrate, was enhanced in combination.

### Example 3-4. Confirmation of GMP Production Ability of Formate Dehydrogenase-Deleted Strain and Formate-Dependent Phosphoribosylglycinamide Formyltransferase-Enhanced Strain

The GMP production ability was confirmed using the XMP culture solutions of the strains prepared in Example 3-1 and Example 302, and control parent strain in the following manner (see Korean Patent Publication No. 10-2011-0105662 A).

Specifically, the strains were cultured using the fermentation titer evaluation method of Example 3-3. After completion of the culture, the XMP (5'-xanthosine monophosphate) production was measured using HPLC.

Thereafter, for conversion of the produced XMP into GMP, the following conversion additives and *E. coli* XMP aminase were added to the flask fermentation solution, and then a conversion reaction was conducted at 40°C for 2.5 hours. As a result of the experiment above, the conversion rate, which accounts for GMP production per consumed XMP, is shown in Table 13.

**[Table 13]**

| Comparison of GMP Production Ability according to Deletion of Formate Dehydrogenase and Enhancement of Formate-Dependent Phosphoribosylglycinamide Formyltransferase | | | |
|---|---|---|---|
| Name of Strain | Concentration (g/L) XMP | Conversion Rate (%) GMP | (GMP produced/XMP consumed) |
| CJX1664 | 4.24 | 3.06 | 72.2 |
| CJX1664_del fdh | 4.38 | 3.17 | 72.4 |
| CJX 1664_Pcj7/purT | 4.37 | 3.16 | 72.2 |
| CJX1664_del fdh-Pcj7/purT | 4.51 | 3.26 | 72.3 |

As shown in Table 13 above, it was confirmed that GMP was produced through a conversion reaction from XMP produced by each strain.

### <Conversion Additive>

phytic acid 1.8 g/L, MgSO₄ 4.8 g/L, nymeen 3 mL/L, xylene 2%, adenine 100 mg/L, Na₂HPO₄ 7.7 g/L, glutamine 2 g/L, glucose 46 g/L

### Example 4. Preparation of Strain with gcvPTH Enhancement, sdaA Deletion, and glyA Enhancement based on Strain with Deletion of Formate Dehydrogenase and Enhancement of Formate-Dependent Phosphoribosylglycinamide Formyltransferase and Evaluation of XMP Production Ability

### Example 4-1. Preparation of XMP-Producing Strain in which gcvPTH Gene is Additionally Enhanced

A strain, in which a copy of *gcvPT* gene was added in the form of a native promoter, was prepared based on the strain with *fdh* deletion and *purT* promoter enhancement.

The pDCM2-Pn/gcvPTH vector prepared in Example 2-1 was transformed into the CJX1664_del fdh-Pcj7/purT strain prepared in Example 3-2 by electroporation, and then the strain with the vector inserted into the chromosome was selected as primary candidates in a selection medium containing 25 mg/L kanamycin. Thereafter, the strain, in which the copy of *gcvPTH* was added, was selected using a primer pair of SEQ ID NOS: 23 and 24 in the strains, in which homologous recombination occurred. The selected strain was named CJI-CJX1664_del fdh-Pcj7/purT-Pn/gcvPTH.

### Example 4-2. Preparation of XMP-Producing Strain Enhanced by Sda Deletion and glyA Addition

Based on the XMP-producing strain introduced with *purT* enhancement and *gcvPTH* enhancement, for the purpose of securing a serine pool that can lead to glycine, a strain, in which the *sdaA,* which is an endogenous gene of *Corynebacterium stationis,* encoding the same was deleted and a copy number of *glyA* gene was added to the corresponding position, was prepared to confirm the effect on XMP production a glycine pool was further enhanced.

The pDCM2-del_sdaA-Pn/glyA vector prepared in Example 2-3 was transformed into the CJX1664_del fdh-Pcj7/purT-Pn/gcvPTH prepared in Example 4-1 by electroporation, and then the strain with the vector inserted into the chromosome was selected as primary candidates in a selection medium containing 25 mg/L kanamycin. Thereafter, the strain, in which the *sdaA* was deleted and the *glyA* gene linked to Pcj7 promoter at the corresponding position was inserted, was selected using a primer pair of SEQ ID NOS: 34 and 35 in the strains, in which homologous recombination occurred. The strain obtained by the method above was named CJX1664_del fdh-Pcj7/purT-Pn/gcvPTH_del sdaA-Pn/glyA.

### Example 4-3. Evaluation of XMP Production Ability of Strain with gcvPTH Enhancement, sdaA Deletion, and glyA Enhancement based on Strain with Deletion of Formate Dehydrogenase and Enhancement of Formate-Dependent Phosphoribosylglycinamide Formyltransferase

The XMP production ability of the strains prepared in Example 4-1 and Example 4-2, and the parent strain was evaluated through flask fermentation titer evaluation in the same manner as in Example 3-3. The above experiment was repeated three times, and the average values of the analysis results are shown in Table 14 below.

**[Table 14]**

| Evaluation of XMP Production Ability of Strain with *sdaA* Deletion, *glyA* Enhancement, and *gcvPTH* Enhancement based on strain with *fdh* deletion and *purT* promoter enhancement | | |
|---|---|---|
| Strain | XMP Concentration (g/L) | XMP Concentration Increase Rate (%) |
| CJX1664 | 4.30 | - |
| CJX1664_del fdh_Pcj7/purT | 4.54 | 5.58% |
| CJX1664_del fdh_Pcj7/purT_Pn/gcvPTH | 4.99 | 16.05% |
| CJX1664_del fdh_Pcj7/purT_Pn/gcvPTH_del sdaA_Pn/glyA | 5.23 | 21.63% |

As shown in Table 14 above, it was confirmed that the XMP production ability of CJX1664_del fdh_Pcj7/purT_Pn/gcvPTH, in which the *gcvPTH* gene was further enhanced compared to the CJX1664_del fdh_Pcj7/purT, was further enhanced, and the CJX1664_del fdh_Pcj7/purT_Pn/gcvPTH_del sdaA_Pn/glyA, which is a strain additionally enhanced by deleting *sdaA* and adding *glyA,* showed increased XMP production. Based on the results, it was confirmed that the XMP production was further enhanced when the *sdaA* was deleted, and *gcvPTH* and *glyA* were enhanced simultaneously with *fdh* deletion and *purT* enhancement.

### Example 4-4. Confirmation of GMP Production Ability of Strain with gcvPTH Enhancement, sdaA Deletion, and glyA Enhancement based on Strain with Deletion of Formate Dehydrogenase and Enhancement of Formate-Dependent Phosphoribosylglycinamide Formyltransferase

The GMP production was confirmed in the same manner as in Example 3-4 using the XMP culture solution of the strains prepared in Example 4-1 and Example 4-2, and the results are shown in Table 15 below.

**[Table 15]**

| Confirmation of GMP Production Ability of Strain with *sdaA* Deletion, *glyA* Enhancement, and *gcvPTH* Enhancement based on strain with *fdh* deletion and *purT* promoter enhancement | | | |
|---|---|---|---|
| Name of Strain | Concentration (g/L) | | Conversion Rate (%) (GMP produced/XMP consumed) |
| | XMP | GMP | |
| CJX1664 | 4.29 | 3.11 | 72.4 |
| CJX1664_del fdh_Pcj7/purT | 4.51 | 3.27 | 72.5 |
| CJX1664_del fdh_Pcj7/purT_Pn/gcvPTH | 5.01 | 3.64 | 72.6 |
| CJX1664_del | 5.22 | 3.78 | 72.4 |
| fdh_Pcj7/purT_Pn/gcvPTH_del | | | |
| sdaA_Pn/glyA | | | |

As shown in Table 15 above, it was confirmed that GMP was produced through a conversion reaction from XMP produced by each strain.

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

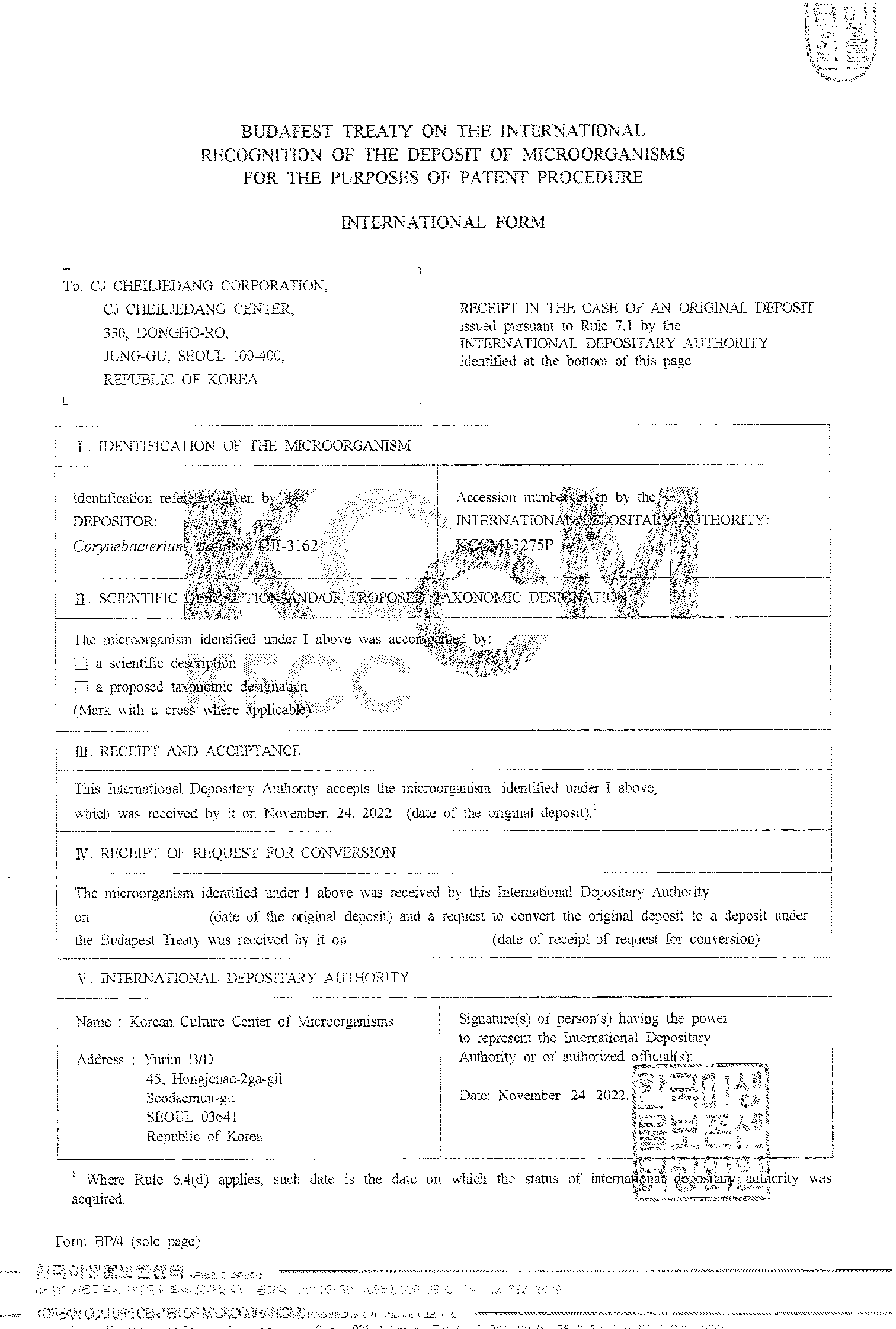

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium stationis CJI-3162 | Accession number given by the
INTERNATIONAL DEPOSITARY AUTHORITY:
KCCM13275P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on November. 24. 2022 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: November. 24. 2022.

¹ Where Rule 6.4(d) applies, such date is the date on which the status of international depositary authority was
acquired.

Form BP/4 (sole page)

한국미생물보존센터 사단법인 한국종균협회
03641 서울특별시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
[?]

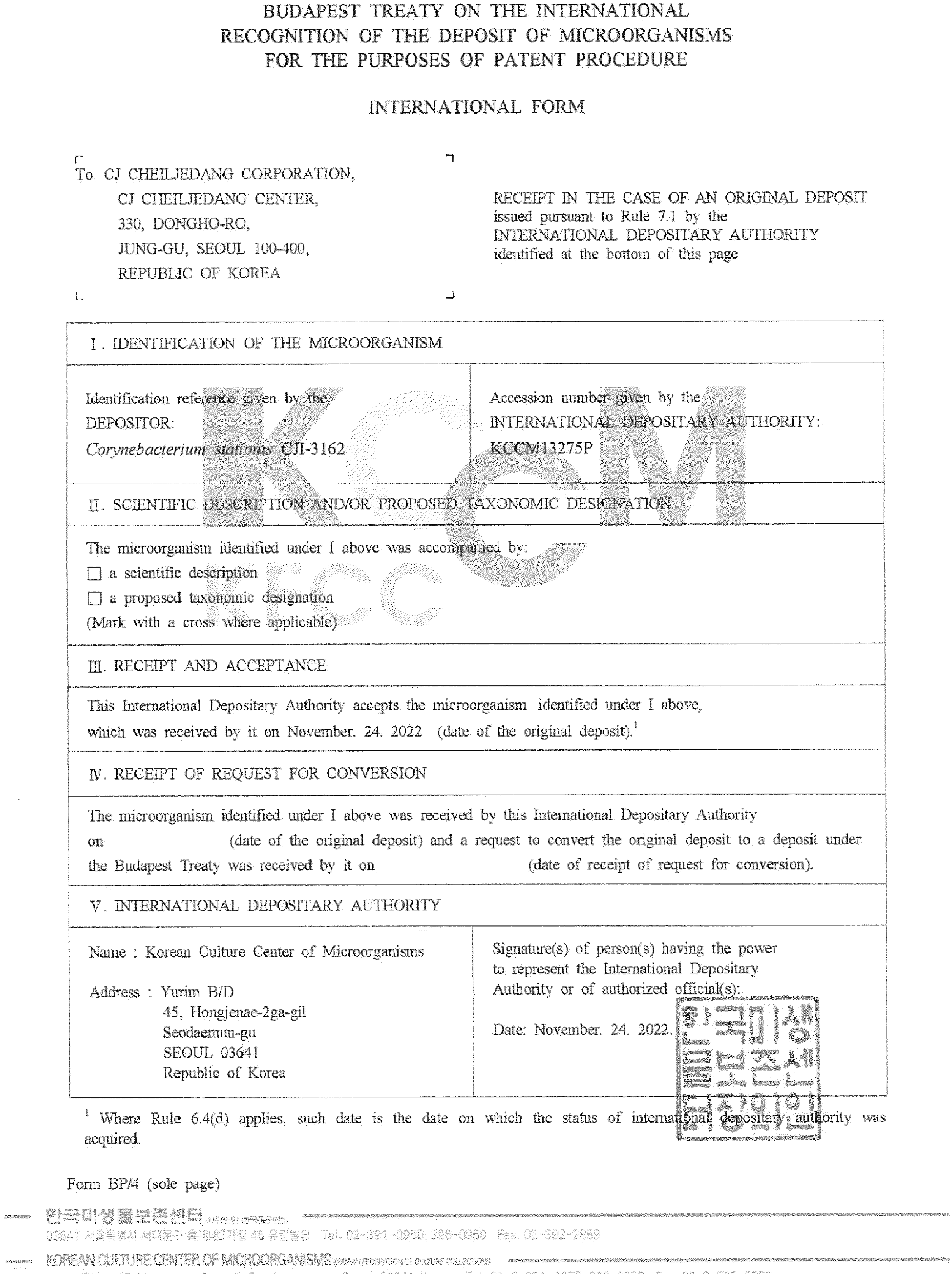
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium stationis CJI-3162 | Accession number given by the
INTERNATIONAL DEPOSITARY AUTHORITY:
KCCM13275P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on November. 24. 2022 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: November. 24. 2022.
¹ Where Rule 6.4(d) applies, such date is the date on which the status of international depositary authority was
acquired.

Form BP/4 (sole page)

한국미생물보존센터 [?]
03641 서울특별시 서대문구 홍제내2가길 45 유림빌딩 Tel. 02-391-0950, 396-0950 Fax: 02-392-2859
KOREAN CULTURE CENTER OF MICROORGANISMS KOREAN FEDERATION OF CULTURE COLLECTIONS
[?]

## Claims

1. A microorganism having a purine nucleotide producing ability, in which the activity of formate dehydrogenase is weakened compared to the endogenous activity thereof.

2. The microorganism of claim 1, wherein the formate dehydrogenase consists of any one or more amino acid sequences selected from the group consisting of SEQ ID NO: 39, SEQ ID NO: 41, SEQ ID NO: 43, and a combination thereof.

3. The microorganism of claim 1, wherein the activity of any one or more proteins selected from the group consisting of (a) to (c) below is further regulated:
(a) the activity of a glycine degradation enzyme is enhanced compared to the endogenous activity thereof;
(b) the activity of serine hydroxymethyltransferase is enhanced compared to the endogenous activity thereof; and
(c) the activity of formate-dependent phosphoribosylglycinamide formyltransferase is enhanced compared to the endogenous activity thereof.

4. The microorganism of claim 1, wherein the microorganism is a microorganism of the genus *Corynebacterium.*

5. The microorganism of claim 4, wherein the microorganism of the genus *Corynebacterium* is *Corynebacterium stationis.*

6. The microorganism of any one claims 1 to 5, wherein the microorganism has an increased purine nucleotide producing ability compared to a non-modified microorganism.

7. A method for producing purine nucleotides, comprising: culturing a microorganism, in which the activity of formate dehydrogenase is weakened compared to the endogenous activity thereof, in a medium.

8. The method of claim 7, wherein the method further comprises recovering a target substance from the cultured microorganism, a culture product of the microorganism, a fermented product of the microorganism, or the culture medium.

9. A composition for producing purine nucleotides: comprising a microorganism, in which the activity of formate dehydrogenase is weakened compared to the endogenous activity thereof, a culture product of the microorganism, a fermented product of the microorganism, or a combination of two or more thereof.

10. Use of a microorganism, in which the activity of formate dehydrogenase is weakened compared to the endogenous activity thereof, for the production of purine nucleotides.
